# EUROPEAN PATENT SPECIFICATION

(11) **EP 1 280 559 B1**
(45) Date of publication and mention of the grant of the patent: **08.03.2006**
(21) Application number: 01928906.5
(22) Date of filing: 26.04.2001
(51) Int. Cl.: A61K 47/48

(54) **ACYLATED CYCLODEXTRIN GUEST INCLUSION COMPLEXES**
ACYLIERTE ZYKLODEXTRIN GUEST-INKLUSION KOMPLEXE
CYCLODEXTRINE ACYLEE: COMPLEXES D'INCLUSION DE MOLECULE INCLUSE

(30) Priority: 11.05.2000 US 203500 P; 19.05.2000 US 205715 P
(43) Date of publication of application: 05.02.2003
(73) Proprietor: EASTMAN CHEMICAL COMPANY, Kingsport, TN 37660 (US)
(72) Inventor: BUCHANAN, Charles, M., Kingsport, Tennessee 37664 (US); SZEJTLI, Jozef, H-1028 Budapest (HU); SZENTE, Lajos, H-1118 Budapest (HU); VIKMON, Maria, H-1026 Budapest (HU); WOOD, Matthew, D., Gray, TN 37615 (US)
(74) Representative: Wibbelmann, Jobst
(86) International application number: PCT/US2001/013499
(87) International publication number: WO 2001/085218

(56) References cited:
- US-A- 4 518 588
- US-A- 4 670 419
- US-A- 5 298 496
- US-A- 5 633 368
- US-A- 5 904 929
- UEKAMA, K. ET AL: "Peracylated beta-cyclodextrins as novel sustained-release carriers for a water-soluble drug, molsidomine" PHARM. PHARMACOL., no. 46, 1994, pages 714-717, XP001080567
- MATSUBARA, K. : "Controlled release of the LHRH agonist buserelin acetate from injectable suspensions containing triacetylated cyclodextrins in an oil vehicle" J. CONTROLLED RELEASE, no. 31, 1994, pages 173-180, XP000458317
- HIRAYAMA, F. ET AL: "Caracterization of peracylated beta-cyclodextrins with different chain lenghts as a novel sustained release carrier for water-soluble drugs" CHEM. PHARM. BULL., vol. 43, no. 1, 1995, pages 130-136, XP001080123 cited in the application

## Description

### Field of Invention

This invention relates to a novel process for the preparation of inclusion complexes comprising acylated cyclodextrin host molecules and guest molecules, a novel process for the preparation of carrier polymer and acylated cyclodextrin:guest molecule inclusion complex composites by melt compounding, novel inclusion complexes comprising acylated cyclodextrins host molecules and guest molecules, novel composites comprising a carrier polymer and an acylated cyclodextrin:guest molecule inclusion complex, shaped articles comprising a carrier polymer and an acylated cyclodextrin:guest molecule inclusion complex capable of the sustained release of guest molecules, and medical devices comprising a carrier polymer and an acylated cyclodextrin:pharmaceutical active inclusion complex capable of the sustained release of guest molecules.

### BACKGROUND

Cyclodextrins (CDs) are cyclic oligomers of glucose which typically contain 6, 7, or 8 glucose monomers joined by α-1,4 linkages. These oligomers are commonly called α-CD, β-CD, and γ-CD, respectively. Higher oligomers containing up to 12 glucose monomers are known but their preparation is more difficult. Each glucose unit has three hydroxyls available at the 2, 3, and 6 positions. Hence, α-CD has 18 hydroxyls or 18 substitution sites available and can have a maximum degree of substitution (DS) of 18. Similarly, β-CD and γ-CD have a maximum DS of 21 and 24 respectively. The DS is often expressed as the average DS, which is the number of substituents divided by the number of glucose monomers in the cyclodextrin. For example, a fully acylated β-CD would have a DS of 21 or an average DS of 3. In terms of nomenclature, this derivative is named heptakis(2,3,6-tri-O-acetyl)-β-cyclodextrin which is typically shortened to triacetyl-β-cyclodextrin.

The production of CD involves first treating starch with an α-amylase to partially lower the molecular weight of the starch followed by treatment with an enzyme known as cyclodextrin glucosyl transferase which forms the cyclic structure. By conducting the reaction in the presence of selected organic compounds, eg. toluene, crystalline CD complexes are formed which facilitate isolation of CD with prcdetermined ring size.

Topologically, CD can be represented as a toroid in which the primary hydroxyls are located on the smaller circumference and the secondary hydroxyls are located on the larger circumference. Because of this arrangement, the interior of the torus is hydrophobic while the exterior is sufficiently hydrophilic to allow the CD to be dissolved in water. This difference between the interior and exterior faces allows the CD or selected CD derivatives to act as a host molecule and to form inclusion complexes with hydrophobic guest molecules provided the guest molecule is of the proper size to fit in the cavity. The CD inclusion complex can then be dissolved in water thereby providing for the introduction of insoluble or sparingly soluble guest molecule into an aqueous environment. This property makes CDs and water soluble CD derivatives particularly useful in the pharmaceutical, cosmetic, and food industries.

Recently, there has been some interest in the development of CD derivatives which could serve as host molecules for hydrophilic guest molecules. The primary interest has been for the sustained release of water soluble drugs. Acylated cyclodextrin derivatives, such as heptakis(2,3,6-tri-O-acetyl)-β-cyclodextrin (Uekama, et al., J. Pharm. Pharmacol. 1994, 46, 714-717), have been proposed as CD derivative host molecules.

In yet another study (Chem. Pharm. Bull. 1995, 43, 130-136), Uekama et al., reported on the preparation and characterization of acylated-β-CDs as a sustained release carrier of different water soluble drugs. The drugs investigated were molsidomine, isosorbide dinitrate, propranolol hydrochloride, and salbutamol sulfate. In still yet another study (Pharm. Sci. 1996,2,533-536), Uekama et al., investigated the controlled release of diltiazem from a combination of short and long chain acylated-β-CDs in dogs.

U.S. Patent No. 5,904,929, to Uekama et al., discloses that a sheet-like or film-like pharmaceutical composition for transmucosal or transdermal administration can be prepared by adding a solution or suspension of C2-C18 acylated cyclodextrins and a drug in an organic solvent onto a backing membrane selected from aluminum foil, polyethylene terephthalate film, or polystyrene film followed by solvent removal. Various drugs are disclosed in this reference, and the preferred peracylated cyclodextrins are the C4-C6 peracylated-β-CD.

### SUMMARY OF INVENTION

The present invention is directed to a method of making an inclusion complex comprising an acylated cyclodextrin host molecule and a guest molecule, wherein the method comprises the steps of: a)contacting the acylated cyclodextrin host molecule and the guest molecule to form an inclusion complex; and b) precipitating the inclusion complex in an aqueous medium.

The present invention is further directed to an inclusion complex comprising an acylated cyclodextrin host molecule and a guest molecule, wherein the guest molecule comprises from about 5 % (wt.) to about 15 % (wt.) of the inclusion complex.

The present invention is in other embodiments related to various inclusion complexes.

Moreover, the present invention relates to a composition comprising a polymer and an inclusion complex, wherein the inclusion complex comprises an acylated cyclodextrin host molecule and a guest molecule. In addition the invention is directed at composites and articles comprising such a composition.

The present invention also is related to a method of making a composition comprising a polymer and an inclusion complex comprised of an acylated cyclodextrin host molecule and a guest molecule, wherein the method comprises: a) contacting the polymer, the acylated cyclodextrin host molecule and the guest molecule to form a polymer/inclusion complex mixture; and b) precipitating the mixture in an aqueous medium.

Additionally, the present invention is related to a method of making a composition comprising a polymer and an inclusion complex comprised of an acylated cyclodextrin host molecule and a guest molecule, wherein the method comprises: a) contacting the polymer, the acylated cyclodextrin host molecule and the guest molecule to form a mixture; and b) melt compounding the mixture to form the composition comprising the polymer and the inclusion complex.

The present invention is further related to a method of making a composition comprising a polymer and an inclusion complex comprised of an acylated cyclodextrin host molecule and a guest molecule, wherein the method comprises: a) contacting the acylated cyclodextrin host molecule and the guest molecule to form an inclusion complex; b) precipitating the inclusion complex in an aqueous medium; c) purifying the inclusion complex to substantially remove the water and any organic solvent; d) contacting the polymer with the purified inclusion complex to form a mixture; and e) melt compounding the mixture to form the composition comprising the polymer and the inclusion complex

Furthermore, the present invention relates to a medical device or a solid pharmaceutical composition comprising a polymer and an inclusion complex, wherein the inclusion complex comprises an acylated cyclodextrin host molecule and a pharmaceutical active guest molecule.

The present invention also relates to a method of making a solid pharmaceutical composition comprising a polymer and an inclusion complex, wherein the inclusion complex comprises an acylated cyclodextrin host molecule and a pharmaceutical active guest molecule, wherein the method comprises:
a) contacting the acylated cyclodextrin host molecule and the pharmaceutical active guest molecule to form an inclusion complex; b) precipitating the inclusion complex in an aqueous medium; c) purifying the inclusion complex to substantially remove the water and any organic solvent; d) contacting the polymer with the purified inclusion complex to form a mixture; and e) melt compounding the mixture to form the composition comprising the polymer and the inclusion complex.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### BRIEF DESCRIPTION OF THE DRAWINGS

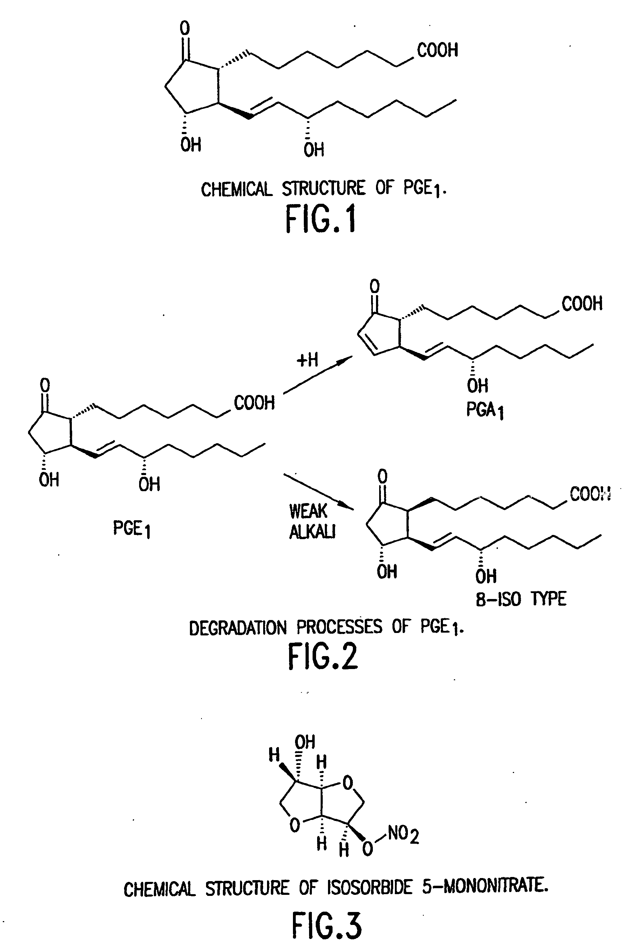

Figure 1 provides the chemical structure of Prostaglandin E₁ (PGE₁).
Figure 2 shows the degradation processes of PGE₁.
Figure 3 provides the chemical structure of isosorbide 5-mononitrate (5-ISMN).
Figure 4 shows the TGA spectrum of an triacetyl-β-CD:Nitroglycerin (NG) complex and a lactose:NG physical mixture.
Figure 5 shows the EGD spectrum of an triacetyl-β-CD:NG complex and a lactose:NG physical mixture.
Figure 6 shows the release profile ofNG from a triacetyl-β-CD:NG complex.
Figure 7 shows the TGA spectrum of triacetyl-β-CD in which 10% weight loss is not observed until 372 °C.
Figure 8 shows the TGA spectrum of triacetyl-β-CD in which the sample was held at 300 °C for 35 minutes.
Figure 9 shows the TGA spectra of (a) triacetyl-β-CD:NG complex, (b) poly(ethylene-co-vinyl acetate), and (c) a composite ofpoly(ethylene-co-vinyl acetate) - triacetyl-β-CD:NG complex.
Figure 10 shows the DSC spectra of (A) a triacetyl-α-CD:5-ISMN complex, (B) a mechanical mixture of triacetyl-α-CD with 5-ISMN, (C) 5-ISMN, and (D) triacetyl-α-CD.
Figure 11 shows the comparison of the release of 5-ISMN from triacetyl-α-CD:5-ISMN and triacetyl-β-CD:5-ISMN inclusion complexes.
Figure 12 shows the TGA spectra of (a) sandawood, (b) triacetyl-β-CD, and (c) a triacetyl-β-CD:sandawood complex.
Figure 13 shows the TGA spectra of (a) Douglas fir, (b) triacetyl-β-CD, and (c) a triacetyl-β-CD:Douglas fir complex.
Figure 14 shows the TGA spectra of filins containing (a) cellulose acetate/20 wt% DEP, (b) cellulose acetate/20 wt% DEP + 10 wt% triacetyl-β-CD, and (c) cellulose acetate/20 wt% DEP + 10 wt% triacetyl-β-CD:sandawood complex.

### DETAILED DESCRIPTION

The present invention may be understood more readily by reference to the following detailed description of preferred embodiments of the invention and the Examples included therein and to the Figures and their previous and following description.

Before the present compounds, compositions, articles, devices, and/or methods are disclosed and described, it is to be understood that this invention is not limited to specific synthetic methods, as such may, of course, vary. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Thus, for example, reference to "an acyl" includes mixtures of acyl groups, reference to "a polymer carrier" includes mixtures of two or more such carriers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

In this specification and in the claims which follow, reference will be made to a number of terms which shall be defined to have the following meanings:

References in the specification and concluding claims to parts by weight, of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included.

By the term "effective amount" of a compound or property as provided herein is meant such amount as is capable of performing the function of the compound or property for which an effective amount is expressed. The exact amount required will vary from process to process, depending on recognized variables such as the compounds employed and the processing conditions observed. Thus, it is not possible to specify an exact "effective amount." However, an appropriate effective amount may be determined by one of ordinary skill in the art using only routine experimentation.

By "pharmaceutically acceptable" is meant a material that is not biologically or otherwise undesirable, i.e., the material may be administered to an individual without causing any undesirable biological effects or interacting in a deleterious manner with any of the other components of the pharmaceutical composition in which it is contained.

By "inclusion complex" is meant a complex or an association between one or more acylated cylodextrin host molecules and one or more guest molecules. That is, the guest molecule may form a complex with the acylated cyclodextrin host molecule by fitting into the cavity of the host. The guest molecule may also form a complex with the acylated cyclodextrin host molecule through association with the outer lip of the cavity or face of the acylated cyclodextrin. Additionally, two or more cyclodextrins, depending upon the molar ratio of guest and host molecules, may form an assembled structure around the guest molecule through association of the faces of the acylated cyclodextrins with the guest molecule.

In the practice of this invention, the useful CDs include those containing from 6-12 unsubstituted glucose monomers and those containing substituents which have a hydroxyl functionality. The preferred unsubstituted cyclodextrins include the α-, β-, and γ-CDs described above. The preferred derivatized CDs containing a hydroxyl functionality include hydroxypropyl CDs, hydroxyethyl CDs, hydroxybutenyl CDs, and sulfobutyl CDs.

Regarding the degree of substitution, the preferred level of substitution is when from about 80% to about 100% of the available hydroxyl groups are acylated. More preferred is when from about 90% to about 100% of the available hydroxyl groups are substituted. Most preferred is when about 100% of the available hydroxyl groups are acylated. It should be understood that the precise DS will depend upon the starting CD.

The preferred acyl groups are those containing from about 1 to about 18 carbon atoms. Specific examples of the preferred acyl groups include formyl, acetyl, propionyl, butyryl, valeryl, hexanoyl, heptanoyl, octanoyl, nonanoyl, decanoyl, undecanoyl, lauryl, tridecanoyl, myristyl, prntadecanoyl, palmityl, heptadecanoyl, stearyl and branched chain acyl groups derived from straight chain acyl. Straight chain acyl groups are preferred. More preferred is when the acyl substituent contains from about 1 to about 4 carbon atoms. Most preferred is when the acyl substituent contains 2 or 3 carbon atoms. Examples of preferred acyl groups include formyl, acetyl, propionyl, and butryl groups.

With regard to incorporation of these acylated CDs into a carrier polymer, it is preferred that the acylated CDs be complexed with a guest molecule after incorporation into the carrier polymer (In this invention, thermoplastic and carrier polymer generally mean the same. It should be noted that in the case of solvent casting of a shaped article, it is not necessary that the carrier polymer also be a thermoplastic carrier polymer.). That is, the acylated CD is initially present in the carrier polymer in the form of an inclusion complex. We have found three distinct methods that permit incorporation of these acylated CD inclusion complexes into the carrier polymer. These methods include melt compounding of the preformed inclusion complex into the carrier polymer, coprecipitation of the inclusion complex with the carrier polymer, and in situ formation of the inclusion complex in the polymer melt.

Incorporation of the inclusion complex by melt compounding requires prior formation of the inclusion complex. To accomplish this goal, we have developed a novel precipitation method for formation of the inclusion complex that is more efficient and which provides for a higher loading of the guest molecule in the host acylated CD relative to other known methods. In the precipitation method, a common solvent for both the acylated CD and the guest molecule is used to dissolve the host acylated CD and guest molecules. For the purpose of this invention, any organic solvent common to the guest and host molecules that has some solubility with water is the preferred organic solvent. Examples of preferred organic solvents include acetone, acetic acid, methyl acetate, ethyl acetate, and ethanol/water with acetone being the most preferred common solvent. Those skilled in the art will recognize that the choice of the solvent will depend upon the structure and solubilities of the guest and host molecule. The combined concentrations of the guest and host molecules in the organic solvent can range from about 1 wt% to about 70 wt%. The preferred concentration is from about 10 wt% to about 50 wt%. Those skilled in the art will also recognize that the host:guest molar ratio in the organic solvent depends upon the host:guest molar ratio of the inclusion complex. This ratio in turn depends upon the structure of the guest molecule and the cavity size of the acylated cyclodextrin. In general, any host:guest molar ratio that provides for effective stablization and sustained release of the guest molecule is preferred. The most preferred host:guest molar ratios are from about 1:1 to about 5:1. After dissolving the guest and host molecules, the mixture is stirred at a temperature ranging form about room temperature to about reflux temperature for about 0.1 to about 6 h. The preferred temperature is from about 25°C to about 50 °C and the preferred mixing time is from about 0.5 to about 2 h. The inclusion complex is isolated by precipitation into water that is at a temperature from about 0 °C to about 30 °C. The precipitation may be rapid, such as from 1 to 300 minutes, more preferably from 5 to 30 minutes. The solid inclusion complex can be isolated and dried by methods well known to those skilled in the art such as filtration to isolate the solid and tray drying to remove excess water or organic solvent.

As noted above, the precipitation method provides for a higher loading of the guest molecule relative to other known methods. The loading can be nominal (just greater than 0%) to 15 wt%. Preferred wt% loading of the guest molecule includes from about 2 wt% to about 15 wt%. The most preferred wt% loading of the guest molecule is from about 5 wt% to about 12 wt%. Of course the precise value will depend upon the molecular weight of the guest compound and the molar ratio of the inclusion complex.

In the case of multicomponent guest molecules such as in fragrances, the precipitation method provides for complexation of the more volatile components. The net result is that the fragrance composition is not altered. Other methods of complex formation gives altered fragrance compositions.

As noted above, the inclusion complex formed by the precipitation method can be incorporated into a carrier polymer by melt compounding. The concentration of the inclusion complex can range from about 0.1 wt% to about 60 wt%. The preferred concentration of the inclusion complex in the carrier polymer is from about 5 wt% to about 25 wt%. The inclusion complex, the carrier polymer, and if desired, other additives, are melt compounded together in a device such as a single or twin screw extruder at a time and temperature suitable to promote mixing. After mixing, the carrier polymer - inclusion complex composite is rapidly cooled. Those skilled in the art will recognize that the time and temperature required for mixing will depend upon factors such as the type of extruder and screw design, the stability of the inclusion complex, and the melt processing temperature of the carrier polymer. Preferably, the processing temperature should be less than that at which the guest molecule is released from the host acylated cyclodextrin. That is, from about 100 °C to about 200 °C. If the host molecule is unusually heat sensitive, a lower processing temperature can be selected. In some cases, it is possible to process above the guest release temperature provided that the processing time at the elevated temperature is brief.

Coprecipitation of the inclusion complex with the carrier polymer is similar to the precipitation method except that the carrier polymer is included in the common organic solvent. That is, a common organic solvent is selected for the acylated CD, the guest molecule, and for the carrier polymer. The carrier polymer is then precipitated from the solution with the inclusion complex distributed through out the carrier polymer. The carrier polymer-inclusion complex composite can then be subsequently thermally processed. Alternative to post precipitation processing, the solution containing the carrier polymer and inclusion complex can be directly cast into an object suitable for direct use. As example of such an object includes cast film. In this case, it is not necessary that the carrier polymer also be a thermoplastic. The temperature, time, concentration, and host: guest molar ratio constraints outlined above apply to coprecipitation as well.

In situ formation of the inclusion complex in the melt optionally involves a device to premix the carrier polymer, the guest molecule, the host molecule, and, if desired, other additives. Suitable devices for premixing include a roll mill, Henschel mixer or ribbon blender. The mixture is then melt compounded together in a device such as a single or twin screw extruder at a time and temperature suitable to promote formation of the inclusion complex and mixing of the inclusion complex with the carrier polymer. After mixing, the carrier polymer-inclusion complex composite is rapidly cooled. The concentration and host:guest molar ratio constraints outlined above also apply in this method as well. Those skilled in the art will recognize that the time and temperature required to promote complex formation and intimate mixing will depend upon factors such as the type of extruder and screw design, the stability and volatility of guest molecule, stability of the complex, and melt processing temperature of the carrier polymer. Preferably, the processing temperature should be less than that at which the guest molecule is released from the host acylated cyclodextrin. That is, from about 100 °C to about 200 °C. If the host molecule is unusually heat sensitive, a lower processing temperature can be selected. In some cases, it is possible to process above the guest release temperature provided that the processing time at the elevated temperature is brief. Without wishing to be bound by theory, it is believed that a high melt viscosity of the carrier polymer matrix can inhibit diffusion of the guest molecule at the higher processing temperatures for a short period of time.

Relative to the inclusion complex, a wide variety of guest molecules can be utilized such as pharmaceutical actives (in this invention, pharmaceutical active and drug active mean the same and are used interchangeably), nutraceuticals, fragrances, plasticizers and insecticides. The guest molecules of the present invention may be hydrophilic or hydrophobic. Preferred guest molecules are pharmaceutical actives and fragrances. Suitable nutraceuticals include acetaldehyde and phytosterols and their derivatives.

Inclusion complexes of the present invention may be comprised of any host molecule herein described and one or more guest molecules, either with or without the carrier polymer. In one embodiment, the present invention relates to inclusion complexes comprising triacetyl-α, β, or γ-cyclodextrin and a non-water soluble or sparingly water soluble guest molecule, such as a non-water soluble pharmaceutical active, a fragrance molecule, a nutraceutical, or an insecticide. Specific embodiments include inclusion complexes comprising triacetyl-α-cyclodextrin and prostaglandin molecules or triacetyl-α or β-cyclodextrin and fragrance molecules. In another embodiment, the present invention relates to inclusion complexes comprising triacetyl-α, β, or γ-cyclodextrin and a water soluble or significantly water soluble guest molecule, such as a water soluble pharmaceutical active, a nutraceutical, or an insecticide. Specific embodiments include inclusion complexes comprising triacetyl-α or β-cyclodextrin and isosorbide-5-mononitrate or triacetyl-β-cyclodextrin and nitroglycerin molecules.

Nonlimiting examples of fragrances include oils of sandalwood, lemon, Douglas fir, patchouli, strawberry, and vanilla. These types of fragrances are available from Aroma Tech (Summerville, NJ). It is well recognized that commercial samples of fragrance oils are actually complex mixtures of many molecules. In this invention, the term fragrance includes both individual molecules and complex mixtures.

A class of particularly preferred pharmaceutical actives are water soluble or sparingly water soluble pharmaceutical actives. In this case, the inclusion complexes provide for the controlled release of the pharmacologically active guest molecule. We have surprisingly found that practice of the above precipitation method for the preparation of the inclusion complex provides an inclusion complex with high loading of the pharmacologically active agent. In many cases, the inclusion complex exhibit sustained and controlled release over several hours. Relative to the prior art, these complexes offer the advantage of sustained availability of the biologically active agent while using smaller amounts of the host molecule. That is, the sustained bioavailability of the pharmacological active agent is increased.

Examples of pharmacologically actives agents include nonsterodial antirheumatic agents, steroids, cardiac glycosides, anticoagulants, benzodiazepine derivatives, benzimidazole derivatives, piperidine derivatives, piperazine derivatives, imidazole derivatives, triazole derivatives, organic nitrates, prostaglandins, and oligionucleotide antisense agents. Nonlimiting examples of preferred pharmacological agents include anti-inflammatory and analgesic agents (eg. acetylsalicylic acid, sodium diclofenac, ibuprofen, sodium naproxen), anticoagulants (heparin, low molecular weight heparins, aspirin, coumadin, dextran, persantine), antidiabetic agents (glibenclamide), antivirals (3TC, AZT, ddC, loviride, indinavir, nelfinavir, tivirapine, ritonavir, squinavir, ddl, ISIS 14803), antistroke agents (lubeluzole, aptiganel, remacemide), vasodilators (glyceryl trinitrate, isosorbide dinitrate, isosorbide 5-mononitrate, pentaerythritol tetranitrate, amyl nitrate, prostaglandin), anticancer agents (ISIS 3521, ISIS 5132), antidepressants (amitriptyline HCl clomipramine HCl, fluoxetine, amoxapine butriptyline HCl), antifungal agents (amphotericin, econazole, flucytosine, miconazole nitrate) and antibacterial agents (amoxicillin, cefaclor, cephalexin, sodium flucloxacillin, lincomycin HCl, clindamycin).

Carrier polymers materials suitable for use with the inclusion complexes include, but are not limited to, polyolefins, aromatic polyesters, vinyl polymers, acrylic polymers, polynitriles, polyamides, aliphatic polyesters, aromatic-aliphatic copolyesters, C1-C10 esters of cellulose, polystyrene, polycarbonate, polylactates, polyanhydrides, polyglycols, polysaccharides, polyhydroxybutyrates, polyhydroxybutyrate-valerate copolymers, polycaprolactone, cellophane, and mixtures thereof. Those skilled in the art will recognize that many of the polymers, such as the aliphatic polyesters, polylactates, or vinyl polymers, are often copolymers containing 2 or more monomer repeat units at varying molar ratios. Preferred thermoplastic materials include polyethylene, polypropylene, polyethylene-propylene copolymers, polyethylene-vinyl acetate copolymers, polyethylene-vinyl alcohol copolymers, polytetrafluoroethylene, starch, cellulose, cellulose acetate, cellulose acetate propionate, cellulose acetate butyrate, cellulose propionate, cellulose butyrate, polylactic acid, polylactic acid-glycolic acid copolymers, polylactic acid-succinic acid copolymers, polyanhydrides, polyvinyl chloride, polystyrene, or mixtures thereof.

Occasionally, thermal processing of these thermoplastic-inclusion complex compositions require the addition of other polymer additives. For example, thermal processing of starch requires the use of water as a plasticizer in order to achieve a thermoplastic, processable starch. Similarly, cellulose esters often require the use of a plasticizer in order to achieve lower melt processing temperatures or certain physical properties. Other components are often added in very small amounts to achieve enhanced thermal stability or to mask taste or odors. Those skilled in the art will recognize when certain polymer additives are necessary and will be able to select those appropriately. In general, polymer additives may be used in the formulations of this invention provided they do not promote instability of the guest molecule or they are not inherently toxic.

If desirable, pharmaceutically acceptable auxiliaries or additives may be added to promote other features such as disintegration, absorption, permeability, or stablization. Examples of such pharmaceutical additives include, but are not limited to, fatty acids, thioglycolates, fatty acid alcohol ester, surfactants, viscosity modifiers, antioxidants, preservatives, inert fillers, or mixtures thereof. Nonlimiting examples are as follows. Fatty acids: oleic acid; thioglycolates: potassium thioglycolate; fatty acid alcohol ester: diisopropyl adapt; surfactants: polyoxyethylene fatty acid ester, fatty acid glycerol esters, alkylpolyglycosides; viscosity modifiers: carboxymethyl cellulose, xanthan gum, methyl cellulose, hydroxypropyl cellulose; antioxidants: ascorbic acid, tocopherol, d-α-tocopheryl polyethylene glycol 1000 succinate; preservatives: scorbic acid; inert fillers: cellulose.

The composites based on a carrier polymer-inclusion complex are capable of highly desirable sustained and controlled release of pharmaceutical active molecules. Hence, those skilled in the art will understand which pharmaceutical active and pharmacologically acceptable molecule to select for treatment.

In the case in which the guest molecule is a pharmaceutical active, the carrier polymer - acylated CD:pharmaceutical active inclusion complex composite can be processed into shaped articles useful as medical devices for the controlled and sustained release of the pharmaceutical active. As nonlimiting examples, a composite based on a carrier polymer - acylated CD:pharmaceutical active inclusion complex can be processed to form a stent or a catheter. Examples in the prior art of stents useful for drug delivery can be found in US 5,980,551 and US 5,383,928. Catheters are thin tubes that are inserted into body cavities and organs. An obvious concern with catheters, particularly those intended for long term use, are secondary infections. Examples of catheters include epidural catheters for providing anesthesia during labor to relieve pain or urinary catheters for treating urinary disjunction that can arise after treatment for diseases such as prostate cancer. Catheters are also used in the treatment of coronary artery disease. One type of coronary angioplasty or PTCA (percutaneous transluminal cornary angioplasty) involves insertion of a catheter into an artery which is then guided to the blocked area of the cornary artery. Once the blockage is located, a smaller balloon tipped catheter is inserted into the existing catheter and guided to the site. Inflation of the balloon causes the artery to stretch and its inner lining to tear at the site so that the narrowing is pressed against the artery wall opening the artery. The balloon is deflated and the catheters are withdrawn. Successful PTCA can significantly improve the health of certain patents. However, there are major complications associated with PTCA: Acute occlusion of the vessel during or after the procedure leading to myocardial infarction and restenosis which leads to a gradual narrowing at the site of the PTCA. For this reason, stents are now becoming the primary form of treatment. A stent is usually a metal device that can be placed in the artery at the site of the dissection. A stent can markedly decrease the incidence of associated myocardial infarction. However, exposure of the stent surface to circulating blood initiates platelet and coagulation reactions that frequently result in thrombus formation and acute thrombosis at the stent site. Generally, the patients are aggressively treated with anticoagulants such as heparin, aspirin, coumadin, dextran, or persantine. Because of the complications associated with systemic anticoagulation, extensive attempts have been made to design a stent that would be non-thrombogenic.

The present invention provides solutions to the above complications. For example, incorporation of an acylated CD:antibiotic inclusion complex directly into a carrier polymer from which the catheter is constructed can provide for the controlled release of the antibiotic which can significantly reduce the number of infections. Incorporation of an acylated CD:anticoagulant dug active inclusion complex into a carrier polymer which is then used to coat the surface of the stent can provide for the controlled and sustained release of the anticoagulant directly at the site which could significantly reduce thrombosis. In the case of stents, other inventors have proposed the use of biodegradable polymers as carriers of drug actives which are used to coat the stent. Biodegradation of the polymer over time releases the drug active by a simple dissolution process. However, this can only be a temporary solution as with time the metal surface of the stent will become exposed. Although such biodegradable polymers can also be used in the present invention as well, the use of a biocompatible and hemocompatible polymer would offer a better solution. In this regard, cellulose acetate is particularly well suited polymer carrier for an acylated CD:anticoagulant drug active inclusion complex.

A polymer carrier containing an acylated CD:pharmaceutical active inclusion complex could be used to construct or coat an implanted medical device could be used to treat other serious diseases. Such devices would be useful in the treatment of diseases that require prolonged intravenous delivery of a drug active. As a nonlimiting example, such devices could be used for the sustained and controlled release of anticancer agents such as antisense oligionucleotides or other anticancer agents such as 5-fluoro-deoxyuridine.

Another nonlimiting example of the use of a polymer carrier containing an acylated CD:pharmaceutical active inclusion complex in the formation of medical devices is use of the composite to form monolithic carrier films useful in a transdermal drug delivery system. The interest in transdermal drug delivery in the medical community arises from the lack of compliance of patients to treatment regimes. Furthermore, it is well understood that oral administration of drugs can lead to digestive difficulties and that the liver can act to remove the drug active. Transdermal drug delivery is viewed as a leading solution to these problems. Transdermal drug delivery patches are similar in appearance to adhesive bandages. When applied to the skin, the transdermal drug delivery patches can dispense the drug active at controlled rates by presenting the drug for absorption through the skin. Many of the current inventions focus on controlling the rate at which the drug is presented to the skin and upon absorption enhancers which increases the transport of the active through the layers of skin. Representative examples of the prior art related to transdermal drug delivery patches can be found in US 5,965,154, US 6,007,837, US 5,989,586, US 6,010,715, and US 6,019,988.

Relative to transdermal drug delivery patches, the acylated CD:drug active inclusion complexes formed by the methods of this invention offer excellent controlled and sustained release of the drug active. The methods for formation of the inclusion complexes described in this invention allows for higher incorporation of the drug active in the acylated CD which in turn provides for higher concentration of the drug active in the transdermal drug delivery patch. These complexes offer increased stability and decreased volatility which allows their incorporation into carrier films by melt extrusion over a wide temperature range or by solvent casting followed by rapid removal of solvent at elevated temperatures. This feature increases the number of drug actives and carrier polymer matrices that can be utilized and reduces concerns about residual solvent. In the present invention, the acylated CD:drug active inclusion complexes are contained in the carrier film and the contact adhesive, permeation enhancer, and the like are applied to the surface of the carrier layer. That is, the drug active is separated from the other components. This provides for enhanced stability of the drug active and increased long term storage.

Another nonlimiting example of the use of a polymer carrier containing an acylated CD:drug active inclusion complex in the formation of medical devices is melt extrusion of the composite to form tablets for controlled and sustained release of the pharmaceutical active. Without attempting to describe all possible formulations, as currently produced, tablets for oral delivery of drug actives are typically comprised of a solid core consisting of a release rate modifier, drug active, and other pharmaceutically acceptable adjuvants or active ingredients. The solid core may optionally be coated where the coating comprises a film coating agent(s) and an optional pore forming agent(s). The coating can serve to further modify the release rate of the drug active. The exact proportion of each ingredient in the tablet are determined by the solubility and chemical properties of the drug active, the chosen dosage, the desired rate of drug delivery, the site where the drug is to be released, and other standard pharmaceutical practices. Generally, the core is formed by compression and, if desired, a coating is applied by coating with a preformed film or by air spraying a coating solution onto the individual tablets. Some of the difficulties associated with this approach to tableting include solubilization and stablization of the drug active, controlled release of the drug active, and delivery of the drug active to the desired site in the intestinal tract. Also, the multistep procedures of combining and mixing multiple ingredients, granulating to achieve a uniform particle size, tablet compression, and coating of the tablet provides a process that is not efficient and economical.

In the present invention, an acylated CD:drug active inclusion complex can be incorporated into a thermoplastic polymer which serves as the matrix for the solid core of the tablet. In certain cases, the acylated CD and the drug active can be added as individual components and complex formation occurs during melt mixing. Other pharmaceutically acceptable ingredients can also be melt compounded into the thermoplastic matrix. If these additives need enhanced stablization or reduced volatility, the additives can be added in the form of inclusion complexes with acylated CDs or other CD derivatives. Following melt compounding, the thermoplastic matrix containing the acylated CD:drug active inclusion complex and additives (thermoplastic matrix core) can be formed into discrete tablets by a number of techniques. For example, the thermoplastic core can be extruded as a rod and chopped into tablets. The preferred method is to extrude the thermoplastic matrix core in the form of a sheet from which individual tablets are stamped or cut. If so desired, the thermoplastic matrix core can be coated by a number of methods. For example, the surface of the extruded sheet can be laminated with a coating film prior to cutting or stamping of the individual tablets. The extruded sheet can also be solvent coated with the coating material. Alternatively, the individual tablets can be coated by air spraying or by passing the tablets through a coating solution.

Melt extrusion of the thermoplastic matrix core offers an efficient alternative to the known methods for tablet production. The use of an extruder to melt mix the tablet components increases the efficiency of the process and provides for a more economical means for preparing solid oral drug formulations. The acylated CD:drug active inclusion complex provides for sustained and controlled release of the drug active decreasing the need for additional release rate modifiers and coating of the tablet. Extrusion to form the thermoplastic matrix core is made possible by the enhanced stability and decreased volatility of the drug active provided by the acylated CD. Water soluble drug actives can be used in these formulations even when water is used to plasticize the thermoplastic during melt compounding and extrusion thus allowing the use of thermoplastic starch in the matrix material for water soluble drug actives. Because the drug active is in the form of an inclusion complex with acylated CDs, the solid oral formulation can be stored for longer periods without degradation of the drug actives or loss of the drug active due to volatility. For example, the acylated CD can provides prolonged shelf life for oral formulations of nitroglycerin.

The compounds of the invention may be conveniently formulated into pharmaceutical compositions composed of one or more of the compounds in association with a pharmaceutically acceptable carrier. See, e.g., *Remington's Pharmaceutical Sciences,* latest edition, by E.W. Martin Mack Pub. Co., Easton, PA, which discloses typical carriers and conventional methods of preparing pharmaceutical compositions that may be used in conjunction with the preparation of formulations of the inventive compounds.

The compounds may be administered orally, parenterally (e.g., intravenously), by intramuscular injection, by intraperitoneal injection, topically, transdermally, implants, or the like, although transdermal or oral administration is preferred. The amount of active compound administered will, of course, be dependent on the subject being treated, the subject's weight, the manner of administration and the judgement of the prescribing physician.

Depending on the intended mode of administration, the pharmaceutical compositions may be in the form of solid, semi-solid or liquid dosage forms, such as, for example, tablets, suppositories, pills, capsules, powders, liquids, suspensions, lotions, creams, gels, or the like, preferably in unit dosage form suitable for single administration of a precise dosage. The compositions will include; as noted above, an effective amount of the selected drug in combination with a pharmaceutically acceptable carrier and, in addition, may include other medicinal agents, pharmaceutical agents, carriers, adjuvants, diluents, etc.

For solid compositions such as tablets, conventional nontoxic solid carriers include, for example, pharmaceutical grades of mannitol, lactose, starch, magnesium stearate, sodium saccharin, talc, cellulose, glucose, sucrose, magnesium carbonate, and the like. Liquid pharmaceutically administrable compositions can, for example, be prepared by dissolving, dispersing, etc., an active compound as described herein and optional pharmaceutical adjuvants in an excipient, such as, for example, water, saline aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example see *Remington's Pharmaceutical Sciences,* referenced above.

For oral administration, fine powders or granules may contain diluting, dispersing, and/or surface active agents, and may be presented in water or in a syrup, in capsules or sachets in the dry state, or in a nonaqueous solution or suspension wherein suspending agents may be included, in tablets wherein binders and lubricants may be included, or in a suspension in water or a syrup. Where desirable or necessary, flavoring, preserving, suspending, thickening, or emulsifying agents may be included. Tablets and granules are preferred oral administration forms, and these may be coated.

Parental administration, if used, is generally characterized by injection. Injectables can be prepared in conventional forms, either as liquid solutions or suspensions, solid forms suitable for solution or suspension in liquid prior to injection, or as emulsions. A more recently revised approach for parental administration involves use of a slow release or sustained release system, such that a constant level of dosage is maintained. See, e.g., U.S. Patent No. 3,710,795.

For topical administration, liquids, suspension, lotions, creams, gels or the like may be used as long as the active compound can be delivered to the surface of the skin.

This invention can be further illustrated by the following examples of preferred embodiments, although it should be understood that these examples are included merely for purposes of illustration and are not intended to limit the scope of the invention unless otherwise specifically indicated. The starting materials are commercially available unless otherwise described. All percentages are by weight unless otherwise described.

The compounds of the invention may be readily synthesized using techniques generally known to those skilled in the art. Suitable experimental methods for making and derivatizing inclusion complexes are described, for example, in the references cited in the Background section herein above, the disclosures of which are hereby incorporated by reference for their general teachings and for their synthesis teachings. Methods for making specific and preferred compounds of the present invention are described in detail in the Examples below.

### Examples

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Example 1 Preparation of triacetyl-β-CD:Nitroglycerin (NG) complexes.

A solution containing 29 g of triacetyl-β-CD (DS = 21) dissolved in 400 mL of 50 % ethanol was prepared by ultrasonication at 35-40 °C. To the triacetyl-β-CD solution was added approximately 3.5 g of NG dissolved in 150 mL ethanol. A clear, homogeneous solution was obtained which became opalescent on cooling. The triacetyl-β-CD:NG inclusion complex was precipitated by adding approximately 300 mL of ice water. The complex was allowed to stand in refrigerator (ca. 5 °C) for 48 hours before filtering and drying to a constant weight at 50 °C in the presence of P₂O₅. This procedure provided 32 g of a triacetyl-β-CD:NG inclusion complex containing 9.73 wt% NG as a white powder. The NG content of the mother liquid was found to be 57 µg/mL. The yield of triacetyl-β-CD:NG inclusion complex was approximately 90 %. In order to evaluate the reproducibility and effect of scaling to larger sizes, this procedure was conducted at four different scales. The results are summarized in Table 4.

**Table 4. Composition of triacetyl-β-CD:NG complexes**

| | Experiments | | | |
|---|---|---|---|---|
| | 1 | 2 | 3 | 4 |
| | (2g) | (10g) | (3g) | (30g)¹ |
| NG content of complex (wt%) | 8.75 | 11.0 | 12.8 | 9.73 |
| Weight loss on drying (%) | 12.0 | 0.4 | 1.0 | 0.68² |
| NG content after heat treatment (wt%) | 9.9 | 11.1 | 13.4 | 9.76³ |

| | | | | |
|---|---|---|---|---|
| 1. Approximate combined weight of NG and triacetyl-β-CD. | | | | |
| 2. Water content by Karl Fischer titration. | | | | |
| 3. Dried for 4 h at 70 °C. | | | | |

In every case, the NG content of the complex was large ranging from about 10 to about 13 wt% NG after drying of the complex. The difference in NG content was a consequence of the amount ofNG used in making the complex rather than loss due to drying.

### Example 2. Weight loss ofNG from triacetyl-β-CD:NG complexes during drying.

Samples of triacetyl-β-CD:NG inclusion complexes, as well as a lactose:NG physical mixture, were placed in individual open vessels in 2 mm layers. The samples were dried at 70 °C. Samples were taken at different time intervals and the NG content of the samples was determined by HPLC. Representative results for one triacetyl-β-CD:NG inclusion complex and the lactose:NG physical mixture is summarized in Table 5.

**Table 5. Loss of NG from a triacetyl-β-CD:NG inclusion complex and a lactose:NG physical mixture after storage at 70 °C.**

| Time | NG content remaining | |
|---|---|---|
| (hours) | Complex | Lactose mixture |
| 0 | 9.73% | 7.2% |
| 4 | 9.76% | 5.2% |
| 10 | 9.67% | 4.3% |

This example demonstrates that NG is not lost from the triacetyl-β-CD:NG inclusion complex even after drying at elevated temperatures for extended times.

### Example 3. Thermal analysis of triacetyl-β-CD:NG inclusion complexes.

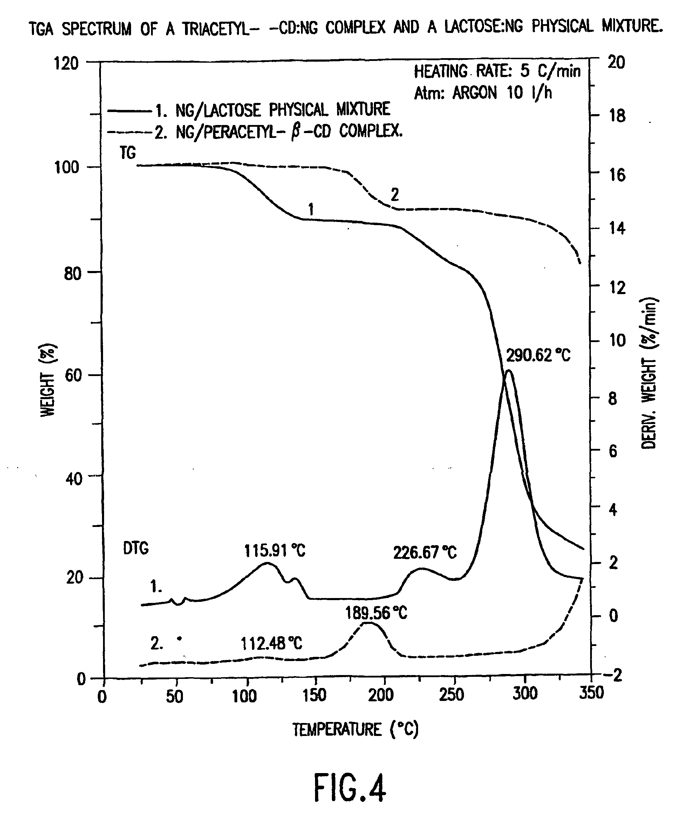

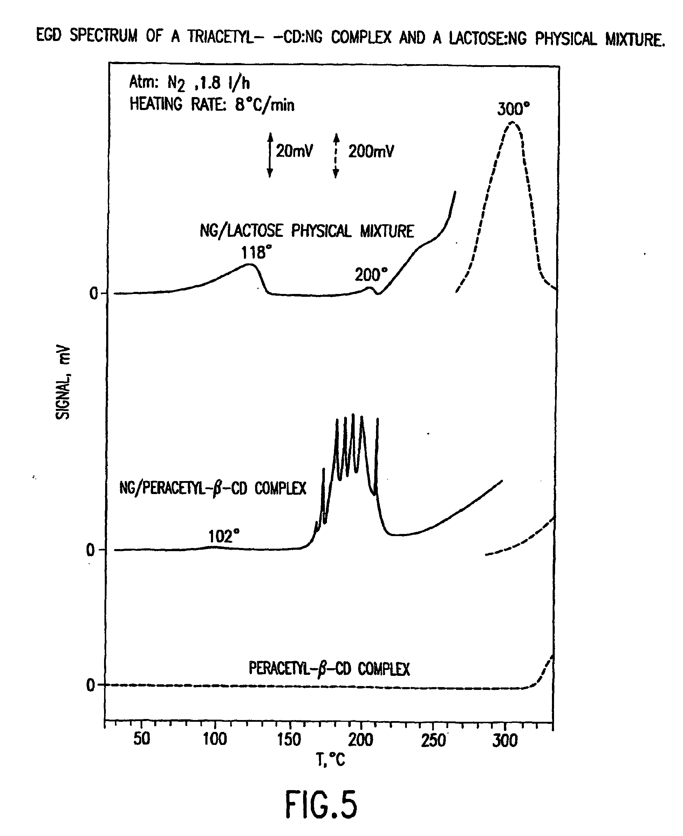

In order to investigate retention of NG upon heating, samples of a triacetyl-β-CD:NG complex (12. 8 % NG) and a lactose:NG physical mixture (7.2 % NG) were analyzed by thermogravimetric analysis (TGA) and by evolved gas detection (EGD). The TGA studies were performed on an Universal V2.3C TA instrument in argon atmosphere, 10 L/h, heating rate of 5 °C/min in a temperature range of 20 - 350 °C. Evolved gas detection curves were taken on a Thermal Analyzer System 916 DuPont (Carle 2000) in a nitrogen atmosphere, 1.8 L/h, heating rate 8 °C/min. The results are summarized in Figures 4 and 5.

In the case of the lactose:NG physical mixture, TGA (Figure 4) shows that the NG is volatilized at about 116 °C. In the case of the triacetyl-β-CD:NG complex, little if any loss of the NG is observed at this temperature. Rather, significant loss ofNG does not occur until approximately 190 °C. Similarly, EGD (Figure 5) shows that the NG is lost in the same temperature range (118 °C) from the lactose:NG physical mixture as that observed in TGA. Likewise, signicant loss ofNG from the triacetyl-β-CD:NG inclusion complex does not occur until about 190 °C. This example illustrates that triacetyl-β-CD acts as a host molecule for NG and provides for significant stablization and reduction of the volatility ofNG.

### Example 4. Release profile of NG from triacetyl-β-CD:NG inclusion complexes.

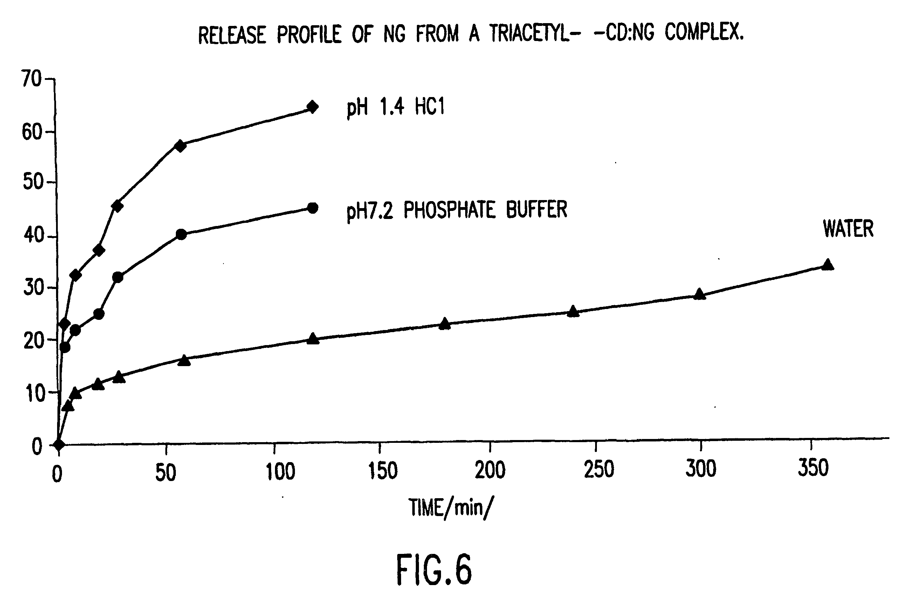

In order to determine the release profile ofNG from triacetyl-β-CD:NG inclusion complexes, the release behavior of NG from a triacetyl-β-CD:NG inclusion complex, a β-CD:NG complex, and lactose:NG physical mixture in different media were investigated. Dissolution tests were carried out by adding samples at concentrations equivalent to 0.1 or 0.5 mg/mL NG to the dissolution media. The media was stirred using magnetic stirrer at approximately 120 r.p.m. at room temperature. At different time intervals, approximately 1 mL of sample was withdrawn, filtered across a membrane filter (0.2 µm), and the filtrates were immediately diluted two-fold with methanol. The amount of dissolved NG was determined by HPLC. Relevant data is summarized in Table 6 and in Figure 6.

**Table 6. Release ofNG in water from a triacetyl-β-CD:NG inclusion complex, a β-CD:NG complex, and lactose:NG physical mixture as a function of time where the compositions were added at concentrations equivalent to 100 µg/mL NG.**

| Time/min. | Dissolved NG µg/mL | | |
|---|---|---|---|
| | Triacetyl-β-CD:NG complex | β-CD:NG complex | Lactose:NG physical mixture |
| 5 | 7.6 | 99.4 | 97.7 |
| 10 | 9.8 | 97.5 | 98.9 |
| 15 | 11.0 | 98.1 | 102.4 |
| 20 | 11.7 | 99.9 | 98.0 |
| 30 | 13.0 | 98.8 | 100.0 |
| 60 | 16.0 | 99.4 | 98.3 |
| 120 | 19.3 | - | - |
| 180 | 21.7 | - | - |
| 240 | 24.0 | - | - |
| 300 | 27.2 | - | - |
| 360 | 32.6 | - | - |
| 1440 | 72.3 | - | - |

As can be seen from Table 6, the release of NG from the β CD:NG complex and the lactose:NG physical mixture in water was very rapid with essentially all of the NG being dissolved in the media within 5 minutes. In contrast, the release ofNG from the triacetyl-β-CD:NG complex was sustained with 1440 min being required for release of 72 µg from the complex. This is further illustrated in Figure 6. When water was the media, approximately 10 µg of NG was release in the first 10 min which followed by a gradual increase of dissolved NG over time. Similar behavior is observed at pH 7.2 and at pH 1.4. However, there is clearly a pH effect. After approximately 180 min, release of 22, 44, and 64 µg ofNG is observed in water, pH 7.2 buffer, and at pH 1.4, respectively. This example illustrates that triacetyl-β-CD:NG complexes provide for sustained release ofNG whereas complexes of β-CD:NG and physical mixtures of lactose:NG do not.

### Example 5. Thermal stability of triacetyl-β-CD.

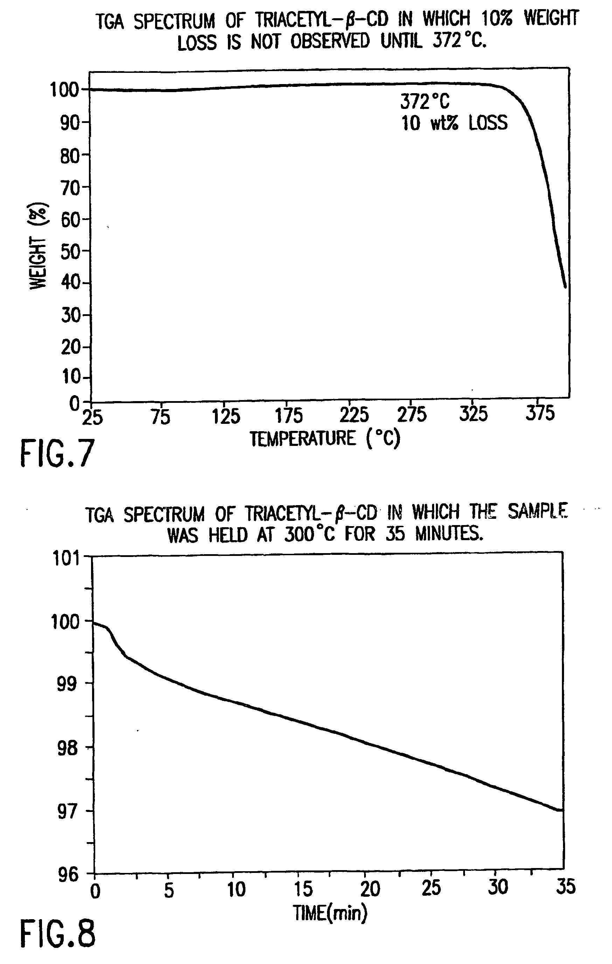

In order to determine the stability of triacetyl-β-CD, a sample was heated to 400 °C at 20 °C/min under nitrogen in a DuPont 2200 thermogravimetric spectrometer (Figure 7). The sample was found to be surprisingly stable with essentially no weight loss (ca. 0.2%) observed until above 325 °C. A sample of triacetyl-β-CD was then held at 300 °C for 35 minutes (Figure 8). Even after extended heating at this temperature, only about 3.2% weight loss was observed. These results demonstrate that triacetyl-β-CD is a surprisingly thermally stable molecule that could act as a thermally stable host molecule for guest molecules such as pharmaceutically active molecules.

### Example 6. Compounding of a triacetyl-β-CD:NG complex in a carrier polymer.

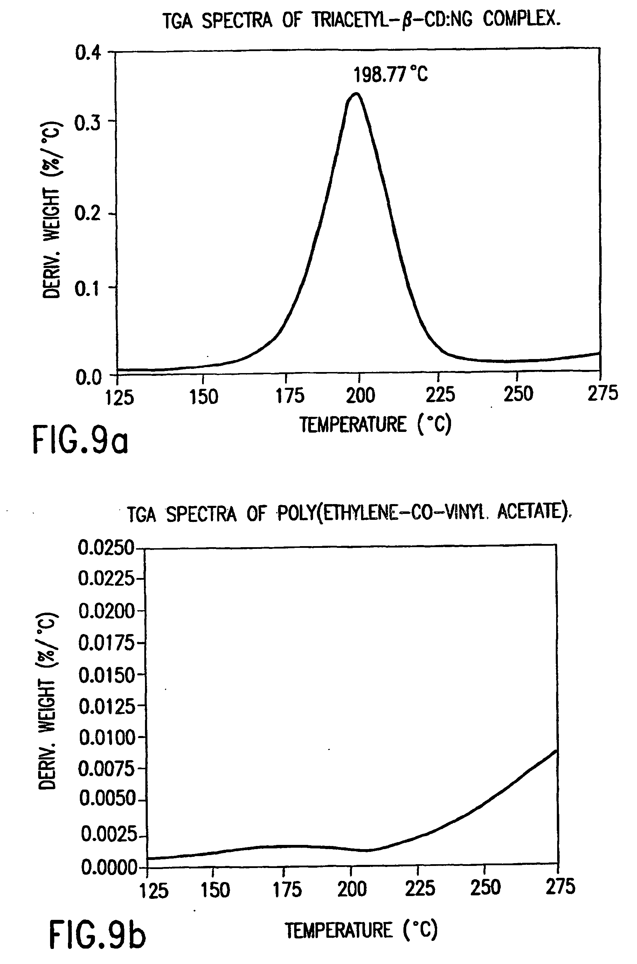

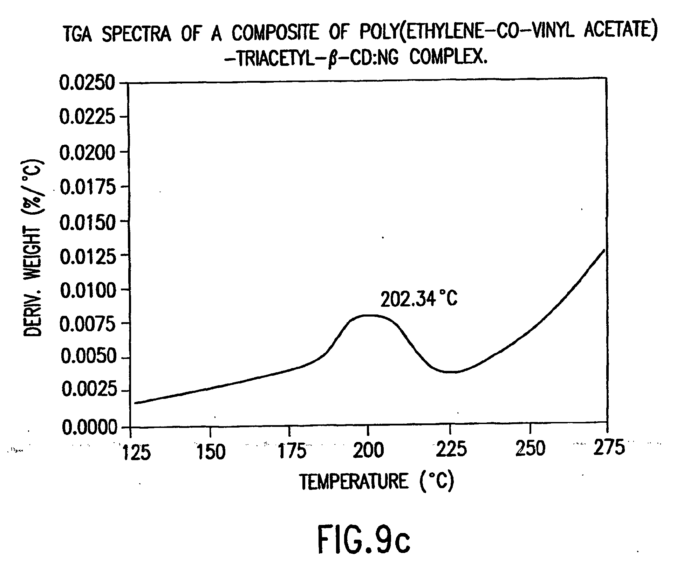

A triacetyl-β-CD:NG complex (1.75 g, 10 wt% NG) and poly(ethylene-co-vinyl acetate) (33.25 g, 25% vinyl acetate) were mixed in a plastic bag. The mixture was then thermally compounded in a Rheometrics Mechanical Spectrometer at 130 °C for 5 minutes. The resulting blend was ground to 5 mm particle size and portions was pressed between two metal plates at 130 °C to produce clear, flexible films containing approximately 0.5 wt % NG. Figure 9 provides TGA spectra of the (a) triacetyl-β-CD:NG complex, (b) poly(ethylene-co-vinyl acetate), and (c) a composite of poly(ethylene-co-vinyl acetate) - triacetyl-β-CD:NG complex. The large peak centered at about 200 °C (Figure 9a) is due to volatilization ofNG which occurs upon reaching the melting point of the complex. As would be expected, a corresponding peak in not present in the poly(ethylene-co-vinyl acetate) (Figure 9b). However, the peak corresponding to volatilization ofNG is clearly present in the spectra of the poly(ethylene-co-vinyl acetate) - triacetyl-β-CD:NG complex. This example demonstrates that a triacetyl-β-CD:pharmaceutical active inclusion complex can be successfully compounded into suitable thermoplastic materials without loss or degradation of the pharmaceutical active. Furthermore, these blends can then be processed into shaped articles such as films, tablets, or other medical devices for extended release of pharmaceutical actives.

### Example 7. Preparation of triacetyl-β-CD:5-ISMN complexes.

Triacetyl-β-CD (15 g) was dissolved in 80 mL of 50 % ethanol by ultrasonication at 50-55°C. Isosorbide 5-mononitrate (2.2 g) dissolved in 20 mL 50 % ethanol was added under continuous stirring. A clear, homogeneous solution was obtained which became opalescent on cooling. The complex was precipitated by adding ice water (approx. 200 mL). The complex was allowed to stand in a refrigerator (ca. 5 °C) for 24 hours. Thereafter, the complex was isolated by filtration and dried to constant weight at 50°C in the presence of P₂O₅. This process provided 15.2 g of the triacetyl-β-CD:5-ISMN complex containing 5.4 wt% of 5-ISMN (determined by capillary electrophoresis) as a white powder.

### Example 8. Preparation of 5-ISMN/triacetyl-α-CD complexes.

Triacetyl-α-CD (15 g) was dissolved in 50 mL of 50 % ethanol by ultrasonication at 70-75 °C. Isosorbide 5-mononitrate (2.3 g) dissolved in 10 mL 50 % ethanol was added under continuous stirring. A clear, homogeneous solution was obtained. The complex was precipitated by adding ice water (approx. 150 mL). The complex was allowed to stand in a refrigerator for 24 hours. Thereafter, the complex was isolated by filtration and dried to constant weight at 50 °C in the presence of P₂O₅. This process provided 13.9 g of the tnacetyl-α-CD:5-ISMN complex containing 5.8 wt% of 5-ISMN (determined by capillary electrophoresis) as a white powder.

### Example 9. Thermal characterization of triacetyl-α-CD:5-ISMN complexes.

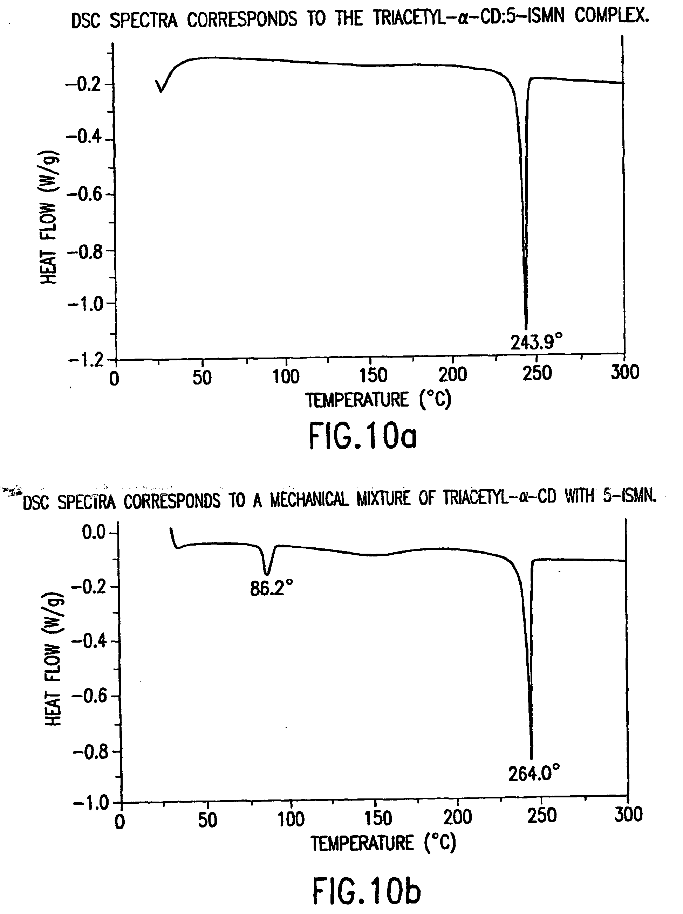

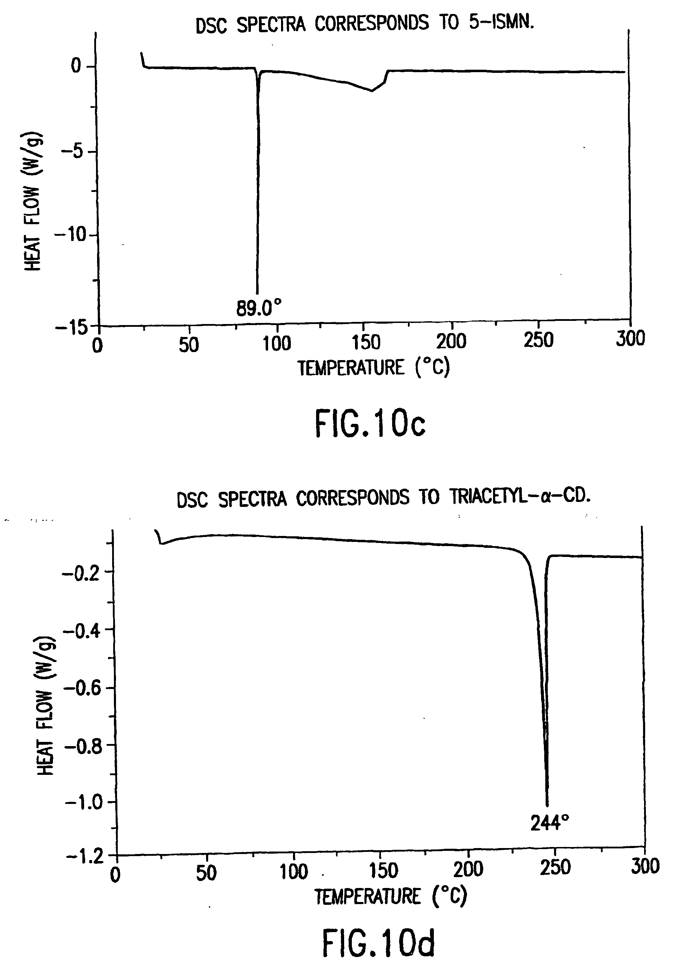

In order to investigate complex formation of 5-ISMN with triacetyl-α-CD, differential scanning calorimetery (DSC) studies were performed on an Universal V2.3C TA instrument in argon atmosphere, 10 L/h, heating rate of 5 °C/min in a temperature range of 20-350 °C. Figure 10 provides the DSC spectra. Figure 10A corresponds to the triacetyl-α-CD:5-ISMN complex, Figure 10B corresponds to a mechanical mixture of triacetyl-α-CD with 5-ISMN, Figure 10C corresponds to 5-ISMN, and Figure 10D corresponds to triacetyl-α-CD. As can be seen, the endotherm due to the melting of 5-ISMN is present in the mechanical mixture but completely absent in the complex. This observation is consistent with a guest/host complex were crystallization and subsequent melting of the 5-ISMN is prohibited due to the association with the triacetyl-α-CD.

### Example 10 Release profile of 5-ISMN from triacetyl-α-CD:5-ISMN and triacetyl-β-CD:5-ISMN complexes.

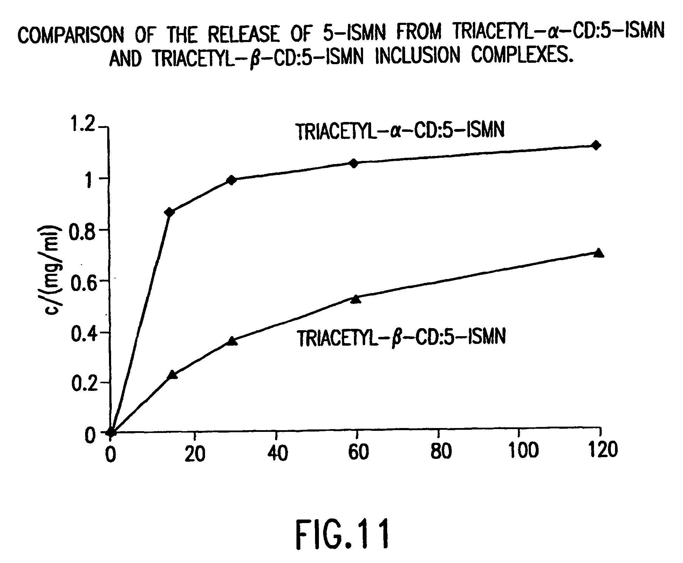

In order to determine the release profile of 5-ISMN from triacetyl-α-CD:5-ISMN and triacetyl-β-CD:5-ISMN complexes, the release behavior of 5-ISMN from the complexes in distilled water were investigated. Dissolution tests were carried out by adding samples at concentrations equivalent to 1.1 or 0.7 mg/mL 5-ISMN to distilled water. The media was stirred using a magnetic stirrer at approximately 120 r.p.m. at room temperature. At different time intervals, approximately 1 mL of sample was withdrawn, filtered through a membrane filter (0.2 µm), and the filtrates were immediately diluted two-fold with 2.5 mM Na₂B₄O₇ pH = 8.0 buffer solution and again by two-fold with methanol. The amount of dissolved 5-ISMN was determined by capillary electrophoresis. Representative data is shown in Table 7 and in Figure 11. As can be seen, the release profiles for the two complexes are different. In the case of the triacetyl-α-CD:5-ISMN complex, nearly all of the 5-ISMN is released in 30-60 minutes. In the case of triacetyl-β-CD:5-ISMN complex, greater than 2 h is required for the release of the 5-ISMN. The release rate difference between the two complexes is likely due to the cavity size difference of the CD derivatives and possibly, due to the hydrophobicity differences of the two CD derivatives.

**Table 7. The release of 5-ISMN from their triacetyl complexes as a function of time.**

| | Dissolved ISMN, mg/mL | | | |
|---|---|---|---|---|
| Time (min) | Triacetyl-β-CD:5-ISMN (5.4 wt% 5-ISMN) 12 mg/mL | Triactyl-β-CD:5-ISMN (5.4 wt% 5-ISMN) 20 mg/mL | triacetyl-β-CD:5-ISMN (2.7 wt% 5-ISMN) 20 mg/mL | triacetyl-α-CD:5-ISMN (5.8 wt% 5-ISMN) 20 mg/mL |
| 15 | 0.1225 | 0.2285 | 0.0398 | 0.8708 |
| 30 | 0.1867 | 0.3583 | 0.0914 | 0.9843 |
| 60 | 0.2940 | 0.5214 | 0.2088 | 1.0434 |
| 120 | 0.4127 | 0.6820 | 0.4578 | 1.0944 |
| 180 | 0.4969 | 0.8100 | 0.5148 | 1.0780 |
| 240 | 0.5926 | 0.9274 | 0.5881 | 1.0747 |
| 300 | 0.6342 | 0.9821 | 0.6468 | 1.0948 |
| 330 | 0.6610 | 1.0015 | - | - |
| 360 | - | - | 0.6507 | 1.0890 |
| 1440 | 0.7023 | 1.0047 | 0.6669 | 0.9903 |

### Example 11. Preparation of triacetyl-α-CD:prostaglandin complexes.

Triacetyl-α-CD (1.7 g) was dissolved in 12 mL of acetone using ultrasonication at room temperature. Three different complexes were prepared by adding prostaglandin (PGE₁) at concentrations of 100 mg, 84 mg, or 35 mg PGE₁ dissolved in 3, 2, 1 mL of acetone, respectively, to the triacetyl-α-CD solutions. Clear, homogeneous solutions were obtained at 1:3, 1:5, and 1:10 host: guest ratios. The solid complexes were obtained by evaporation of the acetone under reduced pressure. The complexes were dried to constant weight in the presence of P₂O₅. The resulting complexes are white powders. PGE₁ and PGA₁ decomposition contents of the complexes were measured by HPLC. For comparative purposes, a mechanical mixture was prepared by wetting 1.7 g of triacetyl-α-CD with 2 mL of ethanol containing 84 mg of dissolved PGE₁. A suspension was obtained and the alcohol was evaporated under reduced pressure. In order to determine the thermal stability of the complexes, the samples were stored at 40 °C for 2 months. Table 8 provides the PGE₁ and PGA₁ content for both the freshly prepared samples and for the aged samples.

**Table 8. PGE₁ and PGA₁ content of freshly prepared complexes and after aging at 40 °C for 2 months.**

| Sample | PGE₁ content (wt %) | | PGA₁ content (wt%) | |
|---|---|---|---|---|
| | As Prepared | Heat Treated | As Prepared | Heat Treated |
| Complex 1:3 | 5.42 | 5.50 | 0.018 | 0.021 |
| Complex 1:5 | 4.40 . | 4.27 | 0.014 | 0.037 |
| Complex 1:10 | 234 | 2.09 | 0.009 | 0.027 |
| Mixture 1:5 | 4.50 | 4.2 | 0.024 | 0.186 |

This example demonstrates the preparation of triacetyl-α-CD complexes with prostaglandin. This example also demonstrates that thermal degradation of PGE₁ is retarded by complexation with triacetyl-α-CD.

### Example 12. Release profile of triacetyl-α-CD:prostaglandin complexes.

Dissolution tests were carried out by adding samples of complexes equivalent to approximately 25, 50 or 100 µg/mL PGE₁/mL to distilled water using magnetic stirring of approximately 120 rpm at room temperature. At different time intervals of stirring, approximately 0.5 mL of sample was withdrawn, filtered through a membrane filter (0.2 µm), and the filtrates were analyzed by HPLC for the determination of dissolved PGE₁. Table 9 provides the percentage of PGE₁ released as a function of time.

**Table 9. Release of PGE₁ from the triacetyl-α-CD complexes as a function of time.**

| | Dissolved PGE₁ (%) | | | |
|---|---|---|---|---|
| Time (min) | complex 1:3 1 mg/mL | complex 1:3 0.5 mg/mL | complex 1:5 1 mg/mL | complex 1:10 1 mg/mL |
| 30 | 25.3 | 28.3 | 58.5 | 23.9 |
| 60 | 47.6 | 43.0 | 74.1 | 50.4 |
| 120 | 66.2 | 70.2 | 88.8 | 55.2 |
| 180 | 78.2 | 78.6 | | 78.4 |
| 240 | 82.5 | 81.6 | 94.2 | 81.0 |
| 360 | 83.7 | 92.7 | 95.6 | 83.5 |
| 1440 | | 99.6 | | |

This example demonstrates that a sustained release of PGE₁ can be achieved with these complexes and that greater than about 6 h is required for complete release of the PGE₁. The release of PGE₁ from the mechanical mixture was much faster with all of the drug being released in 30-60 min. Although not part of this study, it is well known by those skilled in the art that complexes of the parent α-CD with PGE₁ are freely soluble in water and that the drug is released immediately upon dissolving in an aqueous medium.

### Example 13 Preparation of triacetyl-CD:fragrance complexes.

Three different processes were investigated for the preparation of these fragrance complexes. The precipitation method described below is part of the present invention. The other two methods are known in the art and are for comparative purposes.

**Precipitation method.** In this procedure, the triacetyl-CD and the fragrances are dissolved in a suitable solvent which dissolves both components to a similar extent. Acetone, methyl acetate, and ethyl acetate are particularly useful solvents for this purpose, as it dissolves both CDs and fragrances and at the same time does not act as a competitive guest for the triacetyl-CD cavity. Acetone is the particularly preferred solvent An acetone solution of the fragrance and triacetyl-CD is stirred from about 0.5 h to about 3 h at a temperature ranging from ambient to reflux. After stirring for the desired time and temperature, the reaction mixture is poured into water or a water/ground ice mixture with stirring. The triacetyl-CD:fragrance complex is precipitated from the solution. The white precipitate is filtered, washed with cold water, and dried in vacuo to a constant weight. The triacetyl-CD:fragrance formulations were found to have about 8-10% fragrance load values close to that of the expected fragrance content. Moreover, there were no significant alteration observed in the composition of the entrapped fragrances due to the complexation process.

For illustrative purposes, a more detailed process is provided as follows. To a 100 mL double neck glass reactor was added 14.5 g of triacetyl-β-CD and 15 mL of acetone. To the stirred triacetyl-β-CD solution, 1.5 g of vanilla fragrance was added drop-wise without any solvent. The common solution was stirred at room temperature for 1 hour. The reaction mixture was then poured slowly into 700 mL of ground ice/water with intense stirring. The crystalline, white precipitate was stirred for 20 minutes and then allowed to stand at 4 °C overnight. The product was isolated by filtration and dried to constant weight. Yield: 14.9 g solid triacetyl-β-CD:vanilla formulation.

**Kneading Method (Comparative).** In this type of complexation process, the liquid fragrance concentrates and solid triacetyl-CD are thoroughly mixed in the presence of aqueous ethanol. Complexation results from intense kneading. This kind of mechanical activation process has been recommended for complexation of solid or liquid guests with parent cyclodextrins. (Furuta T. et al., Biosci. Biotech. Biochem. 1994, 58, 847; Carli, F. et al., Chimica Oggi. 1987, 3, 61.) The following is representative of a typical procedure: Triacetyl-CD (4.4 g) and about 0.6 gram of lemon or vanilla fragrance (these mass ratios refer to an approximately 1:1 mol/mol ratio) are intensively mixed at ambient temperature in a ceramic mortar together with 2.0 mL of ethanol:water (1:1 by volume) mixture for about 10 minutes. After about 10 minutes, the thoroughly ground reaction mixture becomes a highly viscous paste. Continued mixing for about 20 minutes provides a less viscous paste which can be removed from the reaction vessel. After drying to constant weight, the sample is a hard, coarse solid. The sample can be ground to a powder and sieved to ensure homogeneous particle size. The sieved product appears an easy to handle, free-flowing powder. This process was found to result in a product that had a lower than expected fragrance load of 1-2%, much less than the expected 8-10 % fragrance content. Gas chromatographic studies indicated an altered composition of fragrances in these triacetyl-CD formulations.

**Co-evaporation method (comparative).** A common solution of the triacetyl-CD and the fragrance is evaporated to dryness yielding a dry solid, with loss of the volatile fraction of the surface and complexed fragrance. The key issue in this "common solution" technology is to find the suitable co-solvent The solvents noted earlier will serve this purpose. Other co-solvents were either difficult to remove due to high boiling point or acted as competitive guests thus decreasing the extent of fragrance complexation. By this process, the load of fragrance that was obtained was about 0.5-1.0 % by weight. Moreover, it was also found that this method yields an acetyl-CD:fragrance formulation of an altered fragrance composition.

These three comparative methods show that the precipitation method of the present invention is the most efficient and preferred method for preparation of complexes involving triacetyl cyclodextrins.

### Example 14. Preparation of fragrance/triacetyl-CD complexes.

Using the precipitation method described in example 13, complexes of triacetyl-α-CD and triacetyl-β-CD was prepared with vanilla and lemon oil. The fragrance content of the complexes was determined by gas chromatography (GC). Samples were prepared for GC by dissolving the complexes in dichloromethane at 1 and 10 mg/mL concentrations, respectively, before transferring 1 mL of the solutions into autoinjector vials. A Shimadzu GC 17A Ver. 3 gas chromatograph with advanced flow controller, equipped with Split/Splitless Injection Port Unit was used for the analysis. The carrier gas was nitrogen at a column head-pressure of 65 kPa, and 20 cm/sec linear velocity. Nitrogen make-up gas was used at the FID detector. The detector output was monitored with a DTK personal computer using Class VP Data Handling Software. The column, SBB™-5 (30 m x 0.32 mm x 0.25 µm), was purchased from Supelco. Split injection (20: 1) of 2 µl samples was performed automatically with AOC - 20 Auto Sampler using SGE 10R-S-0.63 syringe. For the lemon complexes, the oven temperature program was as follows: 50 °C for 1 min, increased to 200 °C at 30 °C/min and held for 10 min. The temperature of injector and detector were 220 °C. For the vanilla complexes, the oven temperature program was as follows: 40 °C for 1 min, increased to 200 °C at 50 °C/min and held for 10 min. The temperature of injector and detector were 220 °C. Table 10 provides the fragrance content of the complexes.

**Table 10. The guest content of triacetyl-CD:fragrance complexes.**

| **Sample** | **Fragrance load (wt %) by weight'** |
|---|---|
| triacetyl-α-CD:vanilla | 10.9 |
| triacetyl-β-CD:vanilla | 9.7 |
| triacetyl-α-CD:lemon | 0.7 |
| triacetyl-β-CD:lemon | 6.7 |

| | |
|---|---|
| 1. The values are based upon the mean of three parallel determinations. | |

This examples illustrates that both triacetyl-α-CD and triacetyl-β-CD form complexes with vanilla. In the case of lemon, triacetyl-β-CD is a more suitable host than is triacetyl-α-CD. Furthermore, with the exception of the triacetyl-α-GD:lemon complex, the precipitation method for complex formation provides the expected 8-10 wt% of host material. The low content of lemon in the triacetyl-α-CD complex is likely related to cavity size and restricted guest-host interactions.

### Example 15. Heat stability of triacetyl-CD:fragrance complexes.

In order to determine the heat stability of the triacetyl-CD:fragrance complexes, the complexes were stored at 60 °C for 21 days. Samples were removed at different time periods and analyzed by GC as described in example 14. The results are contained in Table 11.

**Table 11. Weight % of fragrance in triacetyl-CD:fragrance complexes after storage at 60°C.**

| days | triacetyl-α-GD/vanilla | triacetyl-β-CD/vanilla | triacetyl-β-CD/lemon |
|---|---|---|---|
| 0 | 10.9 | 9.7 | 6.7 |
| 2 | 10.6 | 9.4 | 6.7 |
| 6 | 10.2 | 9.0 | 6.0 |
| 14 | 10.0 | 9.0 | 5.8 |
| 21 | 9.8 | 9.1 | 5.8 |

This example demonstrates that these complexes are surprisingly stable with only approximately 10% of the fragrance being lost at 21 days of storage at 60 °C.

### Example 16. Preparation and characterization of carrier polymer - triacetyl-β-CD: fragrance complex composites.

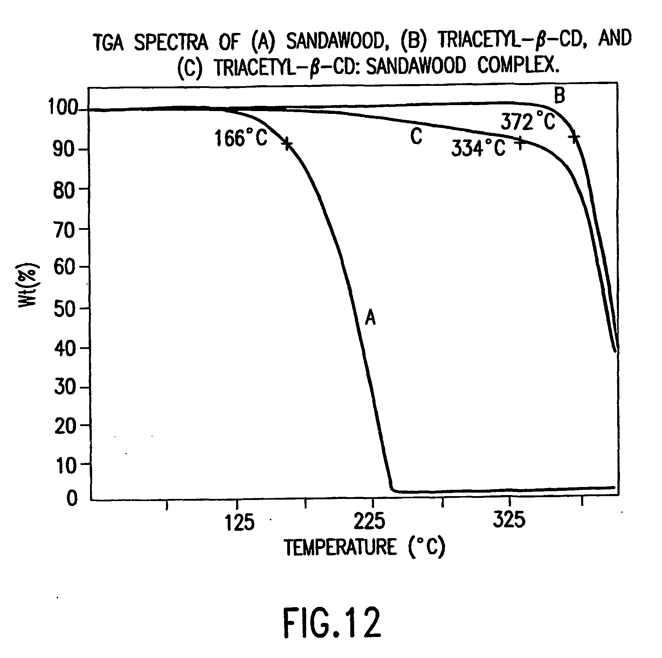

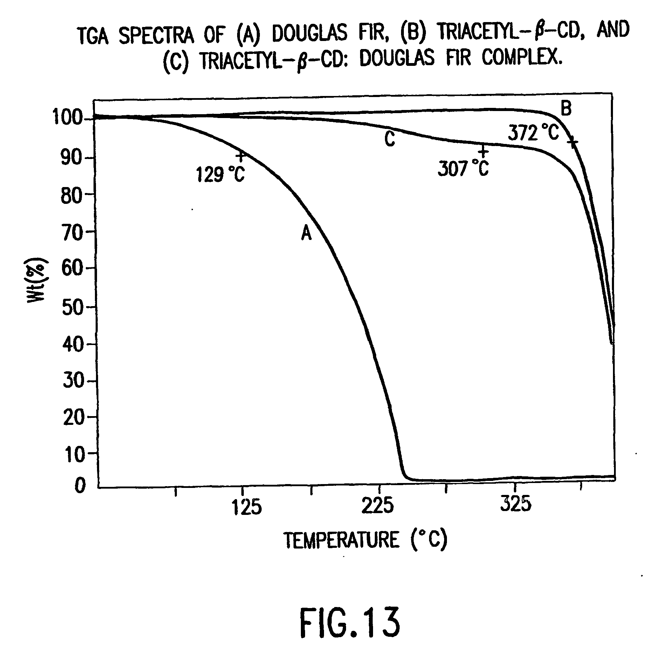

Using the precipitation method described in example 13, complexes of triacetyl-β-CD were prepared with sandalwood and Douglas fir. Using the following general procedure, thermoplastic films containing 10 wt% of each complex was prepared: The complex (3.5 g) containing approximately 10 wt% fragrance, diethylphthalate (7 g, 20 wt%), and cellulose acetate (DS = 2.5) were mixed together in a plastic bag. The mixture was then thermally compounded in a Rheometrics Mechanical Spectrometer at 220 °C for approximately 5 minutes. The resulting blend was ground to 5 mm particle size and portions was pressed between two metal plates at 220 °C to produce clear, flexible, aromatic films containing approximately 1.0 wt % fragrance. For purposes of control samples, films containing no triacetyl-β-CD and films containing triacetyl-β-CD without a host molecule were prepared in which the content of the diethylphthalate was kept at 20 wt%. Figures 12 and 13 provide TGA spectra of triacetyl-β-CD, the free fragrance, and the triacetyl-β-CD:fragrance complex. As illustrated in Figure 12, the sandalwood fragrance begin to volatilize at approximately 125 °C, 10% of the sandalwood was lost at about 166 °C, and all of the sandalwood was gone by about 230 °C. In the case of the complex, volatilization of the sandalwood from the complex did not begin until about 150 °C and volatilization was not complete until about 330 °C. As illustrated in Figure 13, similar behavior was observed with Douglas fir. The Douglas fir fragrance begin to volatilize at approximately 60 °C, 10% of the Douglas fir was lost at about 129 °C, and all of the Douglas fir fragrance was gone by about 230 °C. In the case of the complex, volatilization of the sandalwood from the complex did not begin until about 125 °C and volatilization was not complete until about 305 °C. These complexes provide further evidence of the ability of triacetyl-β-CD to form complexes with guest molecules such as fragrances and their ability to stabilize and reduce volatilization of volatile components.

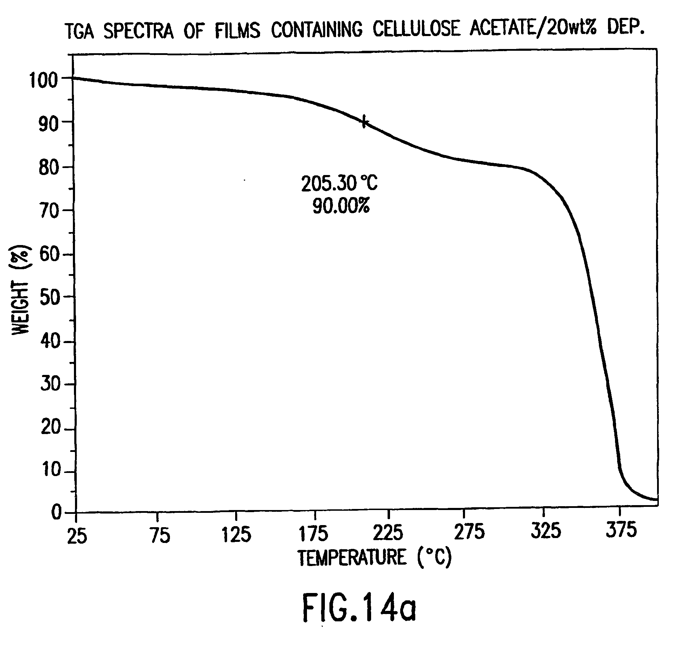

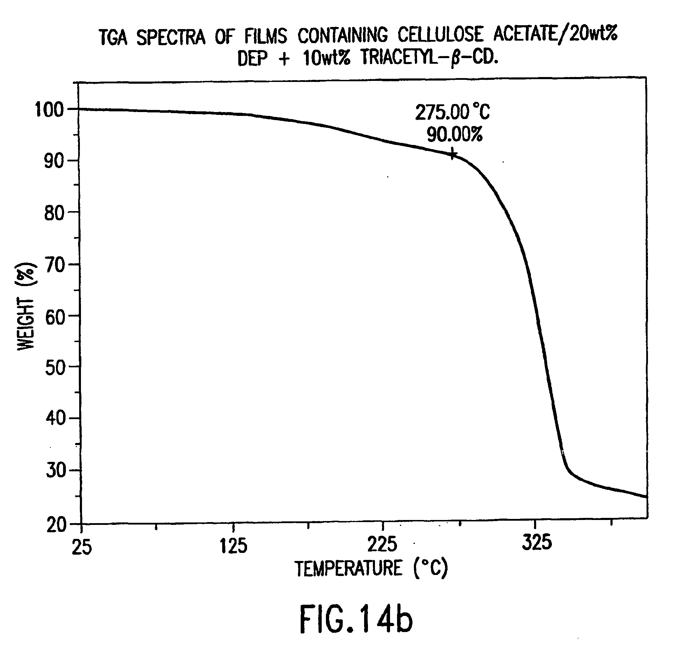

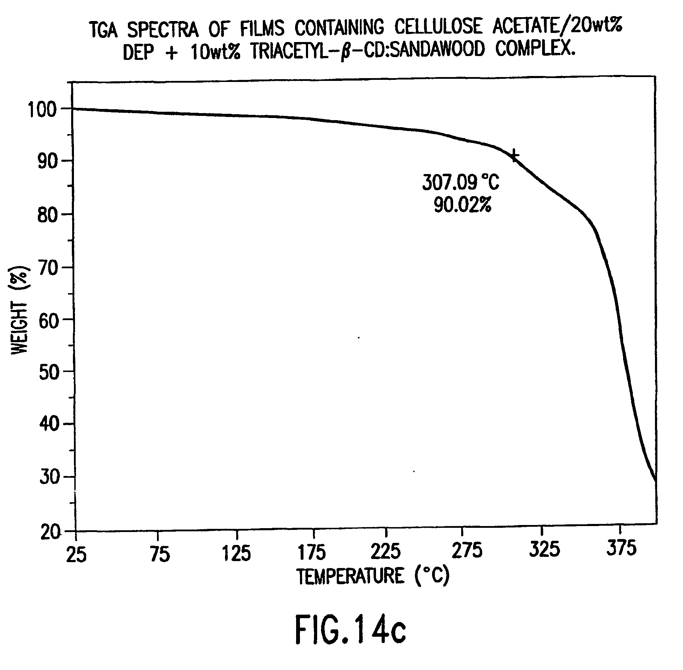

Figure 14 provide TGA spectra of cellulose acetate/diethylphthalate films, cellulose acetate/diethylphthalate films containing 10 wt% of triacetyl-β-CD, and cellulose acetate/diethylphthalate films containing 10 wt% of a triacetyl-β-CD:sandalwood inclusion complex. In the case of the cellulose acetate/diethylphthalate film (Fig 14a), loss of the diethylphthalate is apparent over the temperature range of about 100 °C to about 275 °C. However, in the case of cellulose acetate/diethylphthalate films containing 10 wt% of triacetyl-β-CD (Fig 14 b), loss of diethylphthalate occurs stepwise. There is weight loss of 10 wt% observed between about 75 °C and 275 °C and a second more abrupt loss in weight of approximately 10 wt% between about 275 °C to 300 °C. Without wishing to be bound by theory, the weight loss between 75 °C and 275 °C is due to volatilization of uncomplexed diethylphthalate while that between 275 °C and 300 °C is due to volatilization of complexed diethylphthalate. That is, during the melt processing to form the cellulose acetate/diethylphthalate films containing 10 wt% of triacetyl-β-CD, diethylphthalate forms an inclusion complex With diethylphthalate. In the case of the cellulose acetate/diethylphthalate films containing 10 wt% of triacetyl-β-CD:sandalwood inclusion complex (Fig 14c), a weight loss of approximately 11 wt% is observed between 75 °C and 275 °C which corresponds to loss of the fragrance and loss of uncomplexed diethylphthalate. Again, there is a second more abrupt weight loss which results from disassociation of a triacetyl-β-CD:diethylphthalate inclusion complex and volatilization of the diethylphthalate. These results are illustrative of the complexity of the equilibra that can result by inclusion of a triacetyl-CD:guest molecule complex in a carrier polymer where the composition includes a third component that can also form complexes with the triacetyl-CD. In this case, an inclusion complex forms in situ with diethylphthalate during melt mixing which is in an apparent equilibrium with a triacety-β-CD:fragrance complex. This example demonstrates that inclusion complexes can be formed during melt processing and that, in the present of two or more molecules which can form complexes with the triacetyl-CD, each component can impact the release of the other via a complex equilibra.

## Claims

1. A method of making an inclusion complex comprising an acylated cyclodextrin host molecule and a guest molecule, wherein the method comprises the steps of:
a) contacting the acylated cyclodextrin host molecule and the guest molecule to form an inclusion complex; and
b) precipitating the inclusion complex in an aqueous medium.

2. The method of claim 1, wherein the acylated cyclodextrin host molecule comprises one or more acyl groups containing from 1 to 18 carbon atoms.

3. The method of claim 1, wherein the acylated cyclodextrin host molecule comprises an acylated α-cyclodextrin, a β-cyclodextrin, or a γ-cyclodextrin.

4. The method of claim 1, wherein the acylated cyclodextrin host molecule is 80 % (wt.) to 100 % (wt.) substituted.

5. The method of claim 1, wherein the guest molecule comprises one or more pharmaceutical actives, fragrances, nutraceuticals, plasticizers, or insecticides.

6. The method of claim 1, wherein the guest molecule comprises from 2 % (wt.) to 15 % (wt.) of the inclusion complex.

7. The method of claim 1, further comprising purifying the inclusion complex so that it is substantially free of water and any organic solvent.

8. The method of claim 1, wherein the acylated cyclodextrin host molecule and the guest molecule are contacted in step a) in an organic solvent.

9. The method of claim 8; wherein the acylated cyclodextrin host molecule and the guest molecule are present in the organic solvent from 1 % (wt.) to 70 % (wt.).

10. A composition comprising a carrier polymer and an indusion complex, wherein the indusion complex comprises an acylated cyclodextrin host molecule and a guest molecule, and wherein the inclusion complex is incorporated into the carrier polymer.

11. The composition of claim 10, wherein the polymer comprises one or more polyolefin, aromatic polyester, vinyl polymer, acrylic polymer, polynitrile, polyamide, aliphatic polyester, aromatic-aliphatic copolyester, C1-C10 ester of cellulose, polystyrene, polycarbonate, polylactate, polyanhydride, polyglycol, polysaccharide, polyhydroxybutyrate, polyhydroxybutyrate-valerate copolymer, polycaprolactone, or cellophane.

12. The composition of claim 10, wherein the polymer comprises one or more polyethylene, polypropylene, polyethylene-propylene copolymer, polyethylene-vinyl acetate copolymer, polyethylene-vinyl alcohol copolymer, polytetrafluoroethylene, starch, cellulose, cellulose acetate, cellulose acetate propionate, cellulose acetate butyrate, cellulose propionate, cellulose butyrate, polylactic acid, polylactic acid-glycolic acid copolymer, polylactic acid-succinic acid copolymer, polyanhydride, polyvinyl chloride, or polystyrene.

13. The composition of claim 10, wherein the inclusion complex comprises from 0.1 % (wt.) to 60 % (wt.) of the composition.

14. The composition of claim 10, wherein the acylated cyclodextrin host molecule comprises one or more acyl groups containing from 1 to 18 carbon atoms.

15. The composition of claim 10, wherein the acylated cyclodextrin host molecule comprises an acylated α-cyclodextrin, a β-cyclodextrin, or a γ-cyclodextrin.

16. The composition of claim 10, wherein the acylated cyclodextrin host molecule is 80 % (wt.) to 100 % (wt.) substituted.

17. The composition of claim 10, wherein the guest molecule comprises from 2 % (wt.) to 15 % (wt.) of the inclusion complex.

18. The composition of claim 10, wherein the guest molecule comprises one or more pharmaceutical actives, fragrances, nutraceuticals, plasticizers, or insecticides.

19. The composition of claim 10, wherein the guest molecule comprises a water soluble pharmaceutical active or a significantly water soluble pharmaceutical active.

20. The composition of claim 10, wherein the guest molecule comprises a non-water soluble or sparingly water soluble pharmaceutical active.

21. A method of making the composition of any of claims 10 to 20, wherein the method comprises:
a) contacting the polymer, the acylated cyclodextrin host molecule and the guest molecule to form a mixture; and
b) melt compounding the mixture to form the composition comprising the polymer and the inclusion complex.

22. A method of making the composition of any of claims 10 to 20, wherein the method comprises:
a) contacting the acylated cyclodextrin host molecule and the guest molecule to form an inclusion complex;
b) precipitating the inclusion complex in an aqueous medium;
c) purifying the inclusion complex to substantially remove the water and any organic solvent;
d) contacting the polymer with the purified inclusion complex to form a mixture; and
e) melt compounding the mixture to form the composition comprising the polymer and the inclusion complex.

23. A composite comprising a composition of claims 10-20.

24. A shaped article comprising a composition of claims 10-20.

25. A medical device comprising a composition of claims 10-20.

26. The medical device of daim 25, wherein the medical device is a stent, a catheter, or a transdermal drug delivery patch.

27. A solid pharmaceutical composition comprising a composition of claims 10-20.

## Patentansprüche

1. Ein Verfahren zur Herstellung eines Inklusionskomplexes, umfassend ein acyliertes Cyclodextrin-Wirtsmolekül und ein Gastmolekül, wobei das Verfahren die Schritte umfasst:
(a) Kontaktieren des acylierten Cyclodextrin-Wirtsmoleküls und des Gastmoleküls, um einen Inklusionskomplex zu bilden; und
(b) Ausfällung des Inklusionskomplexes in einem wässerigen Medium.

2. Das Verfahren nach Anspruch 1, wobei das acylierte Cyclodextrin-Wirtsmolekül eine oder mehrere Acylgruppen umfasst, die von 1 bis 18 Kohlenstoffatome enthalten.

3. Das Verfahren nach Anspruch 1, wobei das acylierte Cyclodextrin-Wirtsmolekül ein acyliertes α-Cyclodextrin, ein β-Cyclodextrin oder ein γ-Cyclodextrin umfasst.

4. Das Verfahren nach Anspruch 1, wobei das acylierte Cyclodextrin-Wirtsmolekül 80% (Gew.) bis 100% (Gew.) substituiert ist.

5. Das Verfahren nach Anspruch 1, wobei das Gastmolekül einen oder mehrere pharmazeutische Wirkstoffe, Geruchsstoffe, Nutraceuticals, Weichmacher oder Insektizide umfasst.

6. Das Verfahren nach Anspruch 1, wobei das Gastmolekül von 2% (Gew.) bis 15% (Gew.) des Inklusionskomplexes umfasst.

7. Das Verfahren nach Anspruch 1, das weiterhin die Aufreinigung des Inklusionskomplexes umfasst, so dass dieser im wesentlichen frei von Wasser und beliebigen organischen Lösemitteln ist.

8. Das Verfahren nach Anspruch 1, wobei das acylierte Cyclodextrin-Wirtsmolekül und das Gastmolekül in Schritt a) in einem organischen Lösemittel in Kontakt gebracht werden.

9. Das Verfahren nach Anspruch 8, wobei das acylierte Cyclodextrin-Wirtsmolekül und das Gastmolekül in einem organischen Lösemittel von 1% (Gew.) bis 70% (Gew.) vorhanden sind.

10. Eine Zusammensetzung, umfassend ein Trägerpolymer und einen Inklusionskomplex, wobei der Inklusionskomplex ein acyliertes Cyclodextrin-Wirtsmolekül und ein Gastmolekül umfasst und wobei der Inklusionskomplex in das Trägerpolymer aufgenommen ist.

11. Die Zusammensetzung nach Anspruch 10, wobei das Polymer ein oder mehr Polyolefin, aromatischer Polyester, Vinylpolymer, Acrylpolymer, Polynitril, Polyamid, aliphatischer Polyester, aromatischer/aliphatischer Copolyester, C1-C10-Ester der Cellulose, Polystyren, Polycarbonat, Polylactat, Polyanhydrid, Polyglycol, Polysaccharid, Polyhydroxybutyrat, Polyhydroxybutyrat/Valerat-Copolymer, Polycaprolacton oder Cellophan umfasst.

12. Die Zusammensetzung nach Anspruch 10, wobei das Polymer ein oder mehr Polyethylen, Polypropylen, Polyethylen/Propylen-Copolymer, Polyethylen/Vinylacetat-Copolymer, Polyethylen/Vinylalkohol-Copolymer, Polytetrafluorethylen, Stärke, Cellulose, Celluloseacetat, Celluloseacetatpropionat, Celluloseacetatbutyrat, Cellulosepropionat, Cellulosebutyrat, Polymilchsäure, Polymilchsäure/Glycolsäure-Copolymer, Polymilchsäure/Succinsäure-Copolymer, Polyanhydrid, Polyvinylchlorid oder Polystyren umfasst.

13. Die Zusammensetzung nach Anspruch 10, wobei der Inklusionskomplex von 0,1% (Gew.) bis 60% (Gew.) der Zusammensetzung umfasst.

14. Die Zusammensetzung nach Anspruch 10, wobei das acylierte Cyclodextrin-Wirtsmolekül ein oder mehrere Acylgruppen umfasst, die von 1 bis 18 Kohlenstoffatome enthalten.

15. Die Zusammensetzung nach Anspruch 10, wobei das acylierte Cyclodextrin-Wirtsmolekül ein acyliertes α-Cyclodextrin, ein β-Cyclodextrin oder ein γ-Cyclodextrin umfasst.

16. Die Zusammensetzung nach Anspruch 10, wobei das acylierte Cyclodextrin-Wirtsmolekül 80% (Gew.) bis 100% (Gew.) substituiert ist.

17. Die Zusammensetzung nach Anspruch 10, wobei das Gastmolekül von 2% (Gew.) bis 15% (Gew.) des Inklusionskomplexes umfasst.

18. Die Zusammensetzung nach Anspruch 10, wobei das Gastmolekül einen oder mehrere pharmazeutische Wirkstoffe, Duftstoffe, Nutraceuticals, Weichmacher oder Insektizide umfasst.

19. Die Zusammensetzung nach Anspruch 10, wobei das Gastmolekül einen wasserlöslichen pharmazeutischen Wirkstoff oder einen deutlich wasserlöslichen, pharmazeutischen Wirkstoff umfasst.

20. Die Zusammensetzung nach Anspruch 10, wobei das Gastmolekül einen nicht wasserlöslichen oder kaum wasserlöslichen, pharmazeutischen Wirkstoff umfasst.

21. Ein Verfahren zur Herstellung einer Zusammensetzung nach einem der Ansprüche 10 bis 20, wobei das Verfahren umfasst:
(a) Inkontaktbringen des Polymers, des acylierten Cyclodextrin-Wirtsmoleküls und des Gastmoleküls, um eine Mischung zu bilden; und
(b) Schmelzvermischung der Mischung, um eine Zusammensetzung zu bilden, die das Polymer und den Inklusionskomplex umfasst.

22. Ein Verfahren zur Herstellung einer Zusammensetzung nach einem der Ansprüche 20 bis 20, wobei das Verfahren umfasst:
(a) Inkontaktbringen des acylierten Cyclodextrin-Wirtsmoleküls und des Gastmoleküls, um einen Inklusionskomplex zu bilden;
(b) Ausfällung des Inklusionskomplexes in einem wässerigen Medium;
(c) Reinigung des Inkluxionskomplexes, um im wesentlichen das Wasser und beliebiges organisches Lösemittel zu entfernen;
(d) Inkontaktbringen des Polymers mit dem gereinigten Inklusionskomplex, um eine Mischung zu bilden; und
(e) Schmelzvermischung der Mischung, um eine Zusammensetzung zu bilden, die das Polymer und den Inklusionskomplex umfasst.

23. Ein Komposit, das eine Zusammensetzung nach den Ansprühen 10-20 umfasst.

24. Ein geformter Gegenstand, der eine Zusammensetzung nach den Ansprüchen 10-20 umfasst.

25. Eine medizinische Vorrichtung, die eine Zusammensetzung nach den Ansprüchen 10-20 umfasst.

26. Die medizinische Vorrichtung nach Anspruch 25, wobei die medizinische Vorrichtung ein Stent, ein Katheter oder ein Pflaster für die transdermale Arzneimittelverabreichung ist.

27. Eine feste pharmazeutische Zusammensetzung, die eine Zusammensetzung nach den Ansprüchen 10-20 umfasst.

## Revendications

1. Procédé de fabrication d'un complexe d'inclusions comprenant une molécule hôte de cyclodextrine acylée et une molécule incluse, dans lequel le procédé comprend les étapes consistant à :
a) mettre en contact la molécule hôte de cyclodextrine acylée et la molécule incluse pour former un complexe d'inclusions ; et
b) précipiter le complexe d'inclusions dans un milieu aqueux.

2. Procédé selon la revendication 1, dans lequel la molécule hôte de cyclodextrine acylée comprend un ou plusieurs groupes acyle contenant de 1 à 18 atomes de carbone.

3. Procédé selon la revendication 1, dans lequel la molécule hôte de cyclodextrine acylée comprend une α-cyclodextrine acylée, une β-cyclodextrine ou une γ-cyclodextrine.

4. Procédé selon la revendication 1, dans lequel la molécule hôte de cyclodextrine acylée est substituée à raison de 80 % (en poids) à 100 % (en poids).

5. Procédé selon la revendication 1, dans lequel la molécule hôte comprend un ou plusieurs agents actifs pharmaceutiques, parfums, alicaments, plastifiants ou insecticides.

6. Procédé selon la revendication 1, dans lequel la molécule hôte constitue de 2 % (en poids) à 15 % (en poids) du complexe d'inclusions.

7. Procédé selon la revendication 1, comprenant en outre la purification du complexe d'inclusions de sorte qu'il soit essentiellement dépourvu d'eau et de tout solvant organique.

8. Procédé selon la revendication 1, dans lequel la molécule hôte de cyclodextrine acylée et la molécule incluse sont mises en contact dans l'étape a) dans un solvant organique.

9. Procédé selon la revendication 8, dans lequel la molécule hôte de cyclodextrine acylée et la molécule incluse sont présentes dans le solvant organique à raison de 1 % (en poids) à 70 % (en poids).

10. Composition comprenant un polymère véhicule et un complexe d'inclusions, dans laquelle le complexe d'inclusion comprend une molécule hôte de cyclodextrine acylée et une molécule incluse, et dans laquelle le complexe d'inclusions est incorporé dans le polymère véhicule.

11. Composition selon la revendication 10, dans laquelle le polymère comprend un ou plusieurs composés parmi une polyoléfine, un polyester aromatique, un polymère vinylique, un polymère acrylique, un polynitrile, un polyamide, un polyester aliphatique, un copolyester aromatique-aliphatique, un ester en C1-C10 de cellulose, le polystyrène, le polycarbonate, le polylactate, un polyanhydride, un polyglycol, un polysaccharide, un polyhydroxybutyrate, un copolymère polyhydroxybutyrate-valérate, une polycaprolactone ou la cellophane.

12. Composition selon la revendication 10, dans laquelle le polymère comprend un ou plusieurs composés parmi le polyéthylène, le polypropylène, un copolymère polyéthylène-propylène, un copolymère polyéthylène-acétate de vinyle, un copolymère polyéthylène-alcool vinylique, un polytétrafluoroéthylène, l'amidon, la cellulose, l'acétate de cellulose, l'acétate-propionate de cellulose, l'acétate-butyrate de cellulose, le propionate de cellulose, le butyrate de cellulose, le poly(acide lactique), un copolymère poly(acide lactique)-poly(acide glycolique), un copolymère poly(acide lactique)-poly(acide succinique), un polyanhydride, le poly(chlorure de vinyle) ou le polystyrène.

13. Composition selon la revendication 10, dans laquelle le complexe d'inclusions constitue de 0,1 % (en poids) à 60 % (en poids) de la composition.

14. Composition selon la revendication 10, dans laquelle la molécule hôte de cyclodextrine acylée comprend un ou plusieurs groupes acyle contenant de 1 à 18 atomes de carbone.

15. Composition selon la revendication 10, dans laquelle la molécule hôte de cyclodextrine acylée comprend une α-cyclodextrine acylée, une β-cyclodextrine ou une γ-cyclodextrine.

16. Composition selon la revendication 10, dans laquelle la molécule hôte de cyclodextrine acylée est substituée à raison de 80 % (en poids) à 100 % (en poids).

17. Composition selon la revendication 10, dans laquelle la molécule hôte constitue de 2 % (en poids) à 15 % (en poids) du complexe d'inclusions.

18. Composition selon la revendication 10, dans laquelle la molécule hôte comprend un ou plusieurs agents actifs pharmaceutiques, parfums, alicaments, plastifiants ou insecticides.

19. Composition selon la revendication 10, dans laquelle la molécule hôte comprend un agent actif pharmaceutique soluble dans l'eau ou un agent actif pharmaceutique significativement soluble dans l'eau.

20. Composition selon la revendication 10, dans laquelle la molécule hôte comprend un agent actif pharmaceutique insoluble dans l'eau ou faiblement soluble dans l'eau.

21. Procédé de fabrication de la composition selon l'une quelconque des revendications 10 à 20, dans lequel le procédé comprend :
a) la mise en contact du polymère, de la molécule hôte de cyclodextrine acylée et de la molécule incluse pour former un mélange ; et
b) le mélange en fusion du mélange pour former la composition comprenant le polymère et le complexe d'inclusions.

22. Procédé de fabrication de la composition selon l'une quelconque des revendications 10 à 20, dans lequel le procédé comprend :
a) la mise en contact de la molécule hôte de cyclodextrine acylée et de la molécule incluse pour former un complexe d'inclusions;
b) la précipitation du complexe d'inclusions dans un milieu aqueux ;
c) la purification du complexe d'inclusions pour éliminer substantiellement l'eau et tout solvant organique ;
d) la mise en contact du polymère avec le complexe d'inclusions purifié pour former un mélange ; et
e) le mélange en fusion du mélange pour former la composition comprenant le polymère et le complexe d'inclusions.

23. Composite comprenant une composition selon les revendications 10 à 20.

24. Article façonné comprenant une composition selon les revendications 10 à 20.

25. Dispositif médical comprenant une composition selon les revendications 10 à 20.

26. Dispositif médical selon la revendication 25, dans lequel le dispositif médical est un extenseur, un cathéter ou un timbre de délivrance d'un médicament transdermique.

27. Composition pharmaceutique solide comprenant une composition selon les revendications 10 à 20.
